# EUROPEAN PATENT APPLICATION

(11) **EP 2 279 696 A2**
(43) Date of publication of application: **02.02.2011**
(21) Application number: 10185100.4
(22) Date of filing: 06.10.2005
(51) Int. Cl.: A61B 8/13, A61N 7/02

(54) **Method and system for non-invasive mastopexy**

(30) Priority: 06.10.2004 US 616355 P; 06.10.2004 US 616753 P; 07.10.2004 US 617338 P; 07.10.2004 US 617294 P
(62) Divisional of application: 05805833.0
(71) Applicant: Guided Therapy Systems, L.L.C., Mesa, Arizona 85202-1150 (US)
(72) Inventor: Barthe, Peter G., Phoenix, AZ 85048 (US); Slayton, Michael, H., Tempe, AZ 85283 (US); Makin, Inder, Raj, S., Mesa, AZ 85215 (US)
(74) Representative: Curley, Donnacha John

(57) **Abstract**

Methods and systems for noninvasive mastopexy through deep tissue tightening are provided, comprising a therapeutic ultrasound system configured for providing ultrasound treatment to a deep tissue region, such as a region comprising muscular fascia and ligaments. A method and system for treatment to a deep tissue that contains a lower part of dermis and proximal protrusions of fat labuli into the dermis is also provided, which delivers ultrasound energy to create a thermal injury and coagulating the proximal protrusions of fat labuli, whereby eliminating the fat protrusions into the dermis. A non-invasive method and system for the treatment of blood vessel disorders is provided, wherein a transducer is configured to deliver ultrasound energy to the superficial tissue to treat blood vessels.; Methods and systems for treating stretch marks through deep tissue tightening are provided, comprising a therapeutic ultrasound system configured for providing treatment to a shallow tissue region.

## Description

### Field of Invention

The present invention relates to ultrasound therapy systems, and in particular to a method and system for noninvasive mastopexy, a method and system for treating cellulite, a method and system for treating blood vessel disorders, and a method and system for treating stretch marks.

### Background of the Invention

Coarse sagging of the skin and facial musculature occurs gradually over time due to gravity and chronic changes in connective tissue associated with aging. Invasive surgical treatment to tighten such tissues is common, for example by face-lifts. In these treatments for connective tissue sagging, a portion of the tissue is usually removed, and sutures or other fasteners are used to suspend the sagging tissue structures. On the breasts, the muscular fascia and ligaments form a layer superficial to the muscles and beneath the skin and subcutaneous fat. Breast sagging is due to a process in which the suspensory (Cooper's) ligaments become lax. Surgical tightening of the underlying muscular fascia and ligaments is needed for surgical correction through a procedure referred to as mastopexy, or more commonly known as breast lifts.

Radio frequency (RF) devices have been used to produce heating and shrinkage of skin on the face and breast, with some success as a non-invasive alternative to surgical lifting procedures. However, RF is a dispersive form of energy deposition. It is impossible to control precisely the heated tissue volume and depth, because resistive heating of tissues by RF energy occurs along the entire path of electrical conduction through tissues. Another restriction of RF energy for non-invasive tightening of Cooper's ligaments is unwanted destruction of the overlying fat and skin layers. High impedance to RF within fat, overlying the suspensory connective structures intended for shrinking, leads to higher temperatures in the fat than in the target suspensory structures. Similarly, mid-infrared lasers and other light sources have been used as attempts to non-invasively heat and shrink connective tissues of the dermis. However, light is not capable of non-invasive treatment of Cooper's ligaments, because light does not penetrate deeply enough to produce local heating there. Below a depth of approximately 1 mm, light energy is multiply scattered and cannot be focused to achieve precise local heating.

Cellulite is a common skin disorder that appears as an irregularity of skin contour, often characterized by a dimple appearance of the skin. This condition affects 80% of women worldwide and tends to gather superficially around the thighs, hips, and buttocks.

Cellulite develops in the body when fat is deposited immediately below the dermis and contained in fat chambers (labuli) that can become swollen. As the fat cells grow in size, labuli tend to protrude into a dermis layer, surrounding tissue becomes compressed and hardened, making blood circulation more difficult in trapping fluids. Reduced elasticity of the adipose tissue produces an undesirable tension between the layers. The resulting protrusions and depressions of connective tissue anchor points create the appearance of cellulite.

This condition responds with varying results to invasive procedures, such as liposuction. The non-invasive technologies such as massagers, and low frequency diathermia ultrasound, show marginal results. Preliminary results shown by combination of infrared light and RF energy have some promise of improving skin contours, but significant progress is needed.

Varicose veins (telangiectasia) are the clinical manifestation of underlying venous insufficiency. The venous insufficiency especially in the leg veins allows the venous blood to flow in the retrograde direction in the congested leg veins. The veins eventually dilate due to the increased venous pressure. The aberrant venous flow results in the leg veins from failure of the valves normally present in the veins, as well as the reduced muscle tone of the leg muscles. Further, varicosities of the leg veins result from chronically elevated venous pressure. Venous insufficiency can be present in the superficial or the deep veins, each pathology having its own set of sequelae. Varicose and spider veins are more prevalent in the female population.

Sclerotherapy, laser and intense-pulsed-light therapy, radio-frequency ablation, and surgical extirpation are the modem techniques used to ablate varicosities. During sclerotherapy a sclerosing agent (e.g., polidocanol, hypertonic sodium chloride, etc.) is injected in the dilated vein. A high degree of skill is required for this procedure. The treatment is ineffective in cases where a deeper aberrant vein is missed. Further, the technique has significant morbidity in cases where the agent extravasates outside the blood vessel. Transcutaneous laser or intense pulse light (IPL) are relevant only for small vascular malformations (such as) in the face. However, endovenous laser therapy, whereby a bare fiber is inserted in the varicose vein segment of the vein to coagulate and seal the vein, has proven to be quite effective for veins that are not very deep. The RF-energy-based catheters ablate the vein in a manner similar to the laser devices in coagulating the diseased blood vessel segment. Surgical techniques such as saphenectomy are sometimes used to ligate the dilated part of the veins but can be costly and may cause many complications.

Proliferate disease of the capillary tissue in the facial region also causes hemangionmas and port wine stain defects. These conditions are usually treated with lasers. However, the laser treatments can result in scarring, hyper/hypo pigmentation and other problems after treatment. Thus, more effective and non- invasive methods and systems for treating blood vessel disorders are needed.

Stretch marks, or *striae disease,* are the disfiguring permanent scars left in skin usually caused by excessive stretching such as during and after rapid weight gain or pregnancy. These marks occur in 50 - 90% of all pregnant women, and usually appear in the later half of pregnancy as bright red or purplish lines. While the majority will be on the lower abdomen they can also be found on the thighs, hips, buttocks, breasts and arms of women. During the postpartum period, the reddish lines typically turn into shallow silver scars.

Hydration of the skin via lotions and creams may help reduce the creation of stretch marks and their effects in some cases, but cannot prevent them in women prone to the condition. Studies investigated the effect of applying 0.1 percent tretinoin (retinoic acid or Retin-A) cream to stretch marks *(*S Kang et al. Topical tretinoin (retinoic acid) improves early stretch marks. Arch Dermatol 1996; 132:519-526*.)* Both the length and width of the marks were diminished but side effects include dry and itchy skin and moderate to severe erythema. This treatment works best when applied during the first few days postpartum; however, its effects on breastfeeding are not known. It is toxic and teratogenic, and should never be used during pregnancy.

Postpartum light treatment may be helpful to diminish the appearance of stretch marks. For temporary cosmetic relief, ultraviolet light (UVA) exposure may be used to tan the lighter skin areas represented by stretch marks. In the limited cases where stretch marks are darker than the surrounding skin, intense pulsed light may be used to remove pigment. Pulsed dye lasers are also used.

Patterns of thermal ablation to epidermis and/or dermis and/or fibrous fascia are effective for treatment of various skin conditions. Recently, "fractional photothermolysis" using mid-infrared lasers to produce a microscopic array of thermal injury zones that include both epidermis and dermis was reported to be effective and well-tolerated for treatment of skin remodeling. A primary advantage of fractional photothermolysis is that each zone of thermal injury is smaller than can be easily seen with the unaided eye, and surrounded by a zone of healthy tissue that initiates a rapid healing response. Repeat treatments, which are well tolerated, can be performed until a desired result is obtained. However, similar to any light based treatment, fractional photothermolysis poses the disadvantage that it is intrinsically limited to regions of approximately the upper 1 millimeter of skin, because light that propagates more than about 1 mm through skin has been multiply scattered, and can no longer be focused or delivered effectively to the treatment area. Stretch marks involve both superficial and deep layers of the dermis, as well as fibrous fascia. Therefore it is imperative to treat not only near the surface of skin, but all the way down to the deep dermis and fibrous fascia.

### Summary of the Invention

In accordance with various aspects of the present invention, methods and systems for noninvasive breasts lifts through deep tissue tightening with ultrasound are provided. An exemplary method and system comprise a therapeutic ultrasound system configured for providing ultrasound treatment to a deep tissue region, such as a region comprising muscular fascia and ligaments.

In accordance with various exemplary embodiments, a therapeutic ultrasound system can be configured to achieve depth from 1 mm to 4 cm with a conformal selective deposition of ultrasound energy without damaging an intervening tissue in the range of frequencies from 1 to 15 MHz. In addition, a therapeutic ultrasound can also be configured in combination with ultrasound imaging or imaging/monitoring capabilities, either separately configured with imaging, therapy and monitoring systems or any level of integration thereof.

In accordance with various aspects of the present invention, a method and system for non-invasive treatment of cellulite with ultrasound are provided. An exemplary treatment method and system comprises a therapeutic ultrasound system for providing ultrasound treatment to a deep tissue region that contains a lower part of dermis and proximal protrusions of fat labuli into the dermis. Such an exemplary treatment system delivers conformal ultrasound therapeutic energy to the region creating a thermal injury and coagulating the proximal protrusions of fat labuli, thereby eliminating the fat protrusions into the dermis resulting in improved appearance of the overlaying superficial layers of the skin.

The present invention describes a non-invasive method and system for using ultrasound energy for the treatment of conditions resulting from vascular disorders, such as, for example, in the peripheral extremities and face. Ultrasound energy can be used for treatment of spider veins/engorged veins that are several millimeters in diameter and a up to 70 mm deep, as well to treat other vascular defects in the face and body. In one exemplary embodiment, an image-treatment approach can be used to locate the blood vessel to be treated and then to ablate it non-invasively, while also monitoring the progress of the treatment.

In another embodiment, an ultrasound system and method comprises a transducer and system configured to deliver ultrasound energy to the regions of the superficial tissue (e.g., skin) such that the energy can be deposited at the particular depth at which the vascular malformations (such as but not limited to varicose veins) are located below the skin surface. A method and system for ultrasound treatment of stretch marks are provided. An exemplary method and system are configured for treating stretch marks with therapy only, therapy and monitoring, imaging and therapy, or therapy, imaging, and monitoring using focused, unfocused, or defocused ultrasound at various spatial and temporal energy settings for targeted treatment of stretch marks and surrounding tissues. An exemplary method and system are configured to produce regions of ablation within a treatment zone in spatially defined patterns, rather than heating and destroying the entire volume of the target layer of tissue. Another exemplary method and system are configured to specifically aim such regions of ablation within a treatment zone, to occur at the same location as the stretch marks. An exemplary therapeutic ultrasound system can also be substantially capable of conformal and localized deposition of ultrasound energy as well as targeting and/or monitoring capabilities. Further, a therapeutic ultrasound can also avoid heating, cavitation or other distractive events in the intervening tissue that contains vital structures, as well as tissue posterior to the conformal lesion to avoid the same.

The present application further extends to the following numbered clauses:
1. An ultrasound system configured for noninvasive mastopexy comprising: a control system configured for control of said ultrasound treatment system; an imaging system coupled to said control system, said imaging system configured for imaging of a region of interest, said region of interest comprising a Cooper's ligament; an ultrasound probe configured for generating a conformal lesion within said region of interest to facilitate mastopexy, said control system and said probe being configured for spatial and temporal control to generate said conformal lesion, and said probe configured to operate in a frequency range of about 1 MHz to about 15 MHz.
2. The ultrasound system of clause 1, wherein said ultrasound probe is further configured for spatial and temporal control to generate said conformal lesion.
3. The ultrasound system according to clause 1, wherein said ultrasound probe comprises a transducer, said transducer comprising at least one of a curvilinear array, an annular array, a linear array, and a planar array.
4. The ultrasound system according to clause 1, wherein said ultrasound probe comprises an array and at least two focused transduction elements, wherein said array is at least one of a linear array, a planar array, and annular array.
5. The ultrasound system according to clause 1, wherein said region of interest additionally comprises at least one of muscular fascia, ligaments, suspensory ligaments and a deep tissue region.
6. A method for noninvasive mastopexy, said method comprising: selecting a probe configuration based on a spatial and a temporal parameter; imaging a treatment region comprising at least one of a deep tissue region muscular fascia, ligaments and a Cooper's ligament; verifying said temporal and said spatial parameters of said probe; confirming acoustic coupling of said probe to said treatment region; and applying ultrasound energy to ablate a portion of said treatment region to facilitate mastopexy.
7. The method of clause 6, wherein said step of applying ultrasound energy includes applying conformal ultrasound energy in the range of about 1 MHz to about 15 MHz.
8. An ultrasound system configured for cellulite treatment comprising: a control system configured for control of said ultrasound treatment system; an ultrasound probe configured for generating a conformal lesion within a region of interest, said region of interest comprising at least one of a lower part of dermis, proximal protrusions of fat labuli into said dermis, and a subcutaneous layer to facilitate cellulite treatment; said control system and said probe being configured to operate in a frequency range of about 750 kHz to about 20 MHz.
9. The ultrasound system of clause 8, wherein said ultrasound probe is further configured for spatial and temporal control to generate said conformal lesion.
10. The ultrasound system of clause 8, wherein said conformal lesion provides at least one of physically breaking fat cell clusters and stretching fibrous bonds.
11. The ultrasound system according to clause 8, wherein said transducer probe is further configured to induce at least one of enhancement of lymphatic drainage, evacuation of fat decay products, creation of a thermal injury and coagulation of proximal protrusions of fat labuli.
12. A method for cellulite treatment, said method comprising: selecting a probe configuration based on a spatial and a temporal parameter; verifying said temporal and said spatial parameters of said probe; confirming acoustic coupling of said probe to a treatment region, said treatment region comprising at least one of a lower part of dermis, proximal protrusions of fat labuli into said dermis, and a subcutaneous layer; and applying ultrasound energy to ablate a portion of said treatment region to facilitate cellulite treatment.
13. The method of clause 12, wherein said step of applying ultrasound energy includes applying conformal ultrasound energy in the range of about 750 kHz to about 20 MHz.
14. An ultrasound system configured for treatment of blood vessels comprising: a control system configured for control of said ultrasound treatment system; an imaging system coupled to said control system, said imaging system configured for imaging of a region of interest, said region of interest comprising at least one of spider veins, engorged blood vessels, facial blood vessels, and an occlusion within a blood vessel; an ultrasound probe configured for generating a conformal lesion within said region of interest to facilitate treatment of blood vessel disorders, said control system and said probe being configured to operate in a frequency range of about 2 MHz to about 20 MHz.
15. The ultrasound system of clause 14, wherein said ultrasound probe is configured for at least one of substantial ablation and complete ablation of said region of interest.
16. The ultrasound system according to clause 14, wherein said ultrasound probe is configured to be combined with a pharmaceutical formulation.
17. The ultrasound system according to clause 16, wherein said ultrasound probe and said pharmaceutical formulation are configured to facilitate at least one of increased activity said pharmaceutical formulation, reduced dosage of said pharmaceutical formulation, reduced toxicity of said pharmaceutical formulation, and increased local effect of said pharmaceutical formulation in a site selective manner.
18. The ultrasound treatment system according to clause 14, wherein said treatment system comprises at least two of an imaging system, a therapy system, and a treatment monitoring system, wherein said at least two systems are combined with an auxiliary imaging and treatment monitoring apparatus and a secondary therapy system.
19. The ultrasound treatment system according to clause 18, wherein said auxiliary imaging apparatus comprises at least one of a photographic device and an optical modality.
20. A method for noninvasive treatment of blood vessel disorders, said method comprising: selecting a probe configuration based on a spatial and a temporal parameter; imaging a treatment region comprising at least one of spider veins, engorged blood vessels, facial blood vessels, and an occlusion within a blood vessel; verifying said temporal and said spatial parameters of said probe; confirming acoustic coupling of said probe to said treatment region; and applying ultrasound energy to ablate a portion of said treatment region to facilitate blood vessel treatment.
21. The method of clause 20, wherein said step of applying ultrasound energy includes applying conformal ultrasound energy in the range of about 2 MHz to about 20 MHz.
22. An ultrasound system configured for treating stretch marks comprising: a control system configured for control of said ultrasound treatment system; an imaging system coupled to said control system, said imaging system configured for imaging of a region of interest, said region of interest comprising at least one of an epidermis, a dermis, a deep dermis, and a fibrous fascia; an ultrasound probe configured for generating a conformal lesion within said region of interest to facilitate substantial elimination of stretch marks, said control system and said probe being configured to operate in a frequency range of about 2 MHz to about 50 MHz.
23. The ultrasound system of clause 22, wherein said ultrasound probe is further configured for spatial and temporal control to generate said conformal lesion.
24. The ultrasound system of clause 22, wherein said region of interest ranges in depth from about 0 to about 10 mm.
25. The ultrasound system of clause 22, wherein said ultrasound probe is configured to treat said region of interest, and wherein said region of interest is located at least one of parallel and perpendicular to a stretch mark.
26. The ultrasound system of clause 22, wherein said ultrasound probe is configured to facilitate creation of an anisotropic pattern of tissue damage.
27. The ultrasound system of clause 22, wherein said ultrasound probe is to provide at least two energy effects to a region of interest; wherein said at least two energy effects are configured to facilitate a response in said region of interest, and wherein said at least two energy effects include at least two of thermal, cavitational, hydrodynamic, and resonance induced tissue effects and wherein said response includes at least one of hemostasis, subsequent revascutarization/angiogenesis, growth of interconnective tissue, tissue reformation, ablation of existing tissue, enhanced delivery and activation of medicants, stimulation of protein synthesis and increased cell permeability.
28. The ultrasound treatment system according to clause 22, wherein said control system comprises an imaging system configured for facilitating at least one of one- dimensional imaging, one-dimensional treatment, two-dimensional imaging, two- dimensional treatment, three-dimensional imaging, and three-dimensional treatment.
29. A method for treating stretch marks, said method comprising: selecting a probe configuration based on a spatial and a temporal parameter; imaging a treatment region comprising at least one of an epidermis, a dermis, a deep dermis, and a fibrous fascia; verifying said temporal and said spatial parameters of said probe; confirming acoustic coupling of said probe to said treatment region; and applying ultrasound energy to ablate a portion of said treatment region to facilitate treatment stretch marks.
30. The method of clause 29, wherein said step of applying ultrasound energy includes applying conformal ultrasound energy in the range of about 2 MHz to about 50 MHz.
31. A method for providing noninvasive treatment of blood vessel disorders, said method comprising: localizing of at least one of a spider vein, an engorged blood vessel, a facial blood vessel, and an occlusion within a blood vessel within a region of interest; targeting of delivery of ablative ultrasound energy from a transducer probe to said at least one of said spider vein, said engorged blood vessel, said facial blood vessel, and said occlusion within said blood vessel; and monitoring of results of said targeted delivery within said at least one of said spider vein, said engorged blood vessel, said facial blood vessel, and said occlusion within said blood vessel during and after said targeted delivery to continue planning of treatment.
32. A method for providing treatment of cellulite, said method comprising: localizing of at least one of a lower part of a lower part of a dermis, a proximal protrusion of fat labuli into said dermis, and a subcutaneous layer within a region of interest; targeting of delivery of ablative ultrasound energy from a transducer probe to said at least one of said lower part of said dermis, said proximal protrusion of fat labuli into said dermis, and said subcutaneous layer; and monitoring of results of said targeted delivery within said at least one of said lower part of said dermis, said proximal protrusion of fat labuli into said dermis, and said subcutaneous layer during and after said targeted delivery to continue planning of treatment.
33. A method for providing treatment of stretch marks, said method comprising: localizing of at least one of an epidermis, a dermis, a deep dermis, and a fibrous fascia within a region of interest; targeting of delivery of ablative ultrasound energy from a transducer probe to said at least one of said epidermis, said dermis, said deep dermis, and said fibrous fascia; and monitoring of results of said targeted delivery within said at least one of said epidermis, said dermis, said deep dermis, and said fibrous fascia during and after said targeted delivery to continue planning of treatment.
34. A method for providing noninvasive mastopexy, said method comprising: localizing of at least one of a muscular fascia, a ligament, and a Cooper's ligament within a region of interest; targeting of delivery of ablative ultrasound energy from a transducer probe to said at least one of said muscular fascia, said ligament, and said Cooper's ligament; and monitoring of results of said targeted delivery within said at least one of said muscular fascia, said ligament, and said Cooper's ligament during and after said targeted delivery to continue planning of treatment.

### Brief Description of the Drawings

The subject matter of the invention is particularly pointed out in the concluding portion of the specification. The invention, however, both as to organization and method of operation, may best be understood by reference to the following description taken in conjunction with the accompanying drawing figures, in which like parts may be referred to by like numerals:
FIG. 1 illustrates a block diagram of an exemplary ultrasound treatment system for cosmetic enhancement in accordance with an exemplary embodiment of the present invention;
FIGS. 2A-2F illustrate a cross sectional diagrams of exemplary probe systems in accordance with exemplary embodiments of the present invention;
FIGS. 3A and 3B illustrate block diagrams of an exemplary control system in accordance with exemplary embodiments of the present invention;
FIGS. 4A and 4B illustrate block diagrams of an exemplary probe system in accordance with exemplary embodiments of the present invention;
FIG. 5 illustrates a cross-sectional diagram of an exemplary transducer in accordance with an exemplary embodiment of the present invention;
FIGS. 6A and 6B illustrate cross-sectional diagrams of an exemplary transducer in accordance with exemplary embodiments of the present invention;
FIG. 7 illustrates exemplary transducer configurations for ultrasound treatment in accordance with various exemplary embodiments of the present invention;
FIGS. 8A and 8B illustrate cross-sectional diagrams of an exemplary transducer in accordance with another exemplary embodiment of the present invention;
FIG. 9 illustrates an exemplary transducer configured as a two-dimensional array for ultrasound treatment in accordance with an exemplary embodiment of the present invention;
FIGS. 10A-10F illustrate cross-sectional diagrams of exemplary transducers in accordance with other exemplary embodiments of the present invention;
FIG. 11 illustrates a schematic diagram of an acoustic coupling and cooling system in accordance with an exemplary embodiment of the present invention;
FIG. 12 illustrates a block diagram of a treatment system comprising an ultrasound treatment subsystem combined with additional subsystems and methods of treatment monitoring and/or treatment imaging as well as a secondary treatment subsystem in accordance with an exemplary embodiment of the present invention; and
FIGS. 13A and 13B illustrate schematic diagrams of treatment regions in accordance with exemplary embodiments of the present invention.

### Detailed Description

The present invention may be described herein in terms of various functional components and processing steps. It should be appreciated that such components and steps may be realized by any number of hardware components configured to perform the specified functions. For example, the present invention may employ various medical treatment devices, visual imaging and display devices, input terminals and the like, which may carry out a variety of functions under the control of one or more control systems or other control devices. In addition, the present invention may be practiced in any number of medical contexts and that the exemplary embodiments relating to cosmetic enhancement treatment systems, imaging systems and monitoring systems as described herein are merely indicative of exemplary applications for the invention. For example, the principles, features and methods discussed may be applied to any medical application. Further, various aspects of the present invention may be suitably applied to other applications.

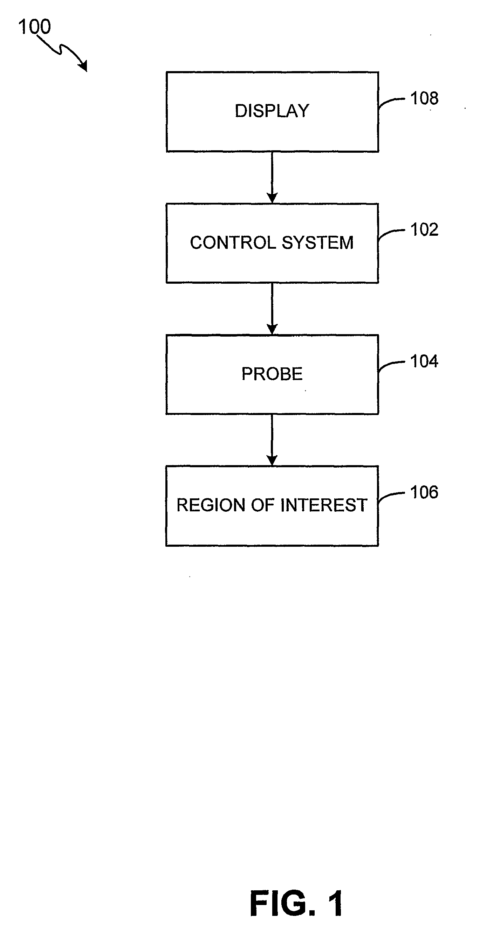

In accordance with various aspects of the present invention, methods and systems for cosmetic enhancement through ultrasound ablation are provided. For example, in accordance with an exemplary embodiment, with reference to FIG. 1, an exemplary treatment system 100 configured to treat a region of interest 106 comprises a control system 102, an imaging/therapy probe with acoustic coupling 104, and a display system 108.

Control system 102 and display system 108 can comprise various configurations for controlling probe 104 and overall system 100 functionality, such as, for example, a microprocessor with software and a plurality of input/output devices, system and devices for controlling electronic and/or mechanical scanning and/or multiplexing of transducers, a system for power delivery, systems for monitoring, systems for sensing the spatial position of the probe and/or transducers, and/or systems for handling user input and recording treatment results, among others. Imaging/therapy probe 104 can comprise various probe and/or transducer configurations. For example, probe 104 can be configured for a combined dual-mode imaging/therapy transducer, coupled or co-housed imaging/therapy transducers, or simply a separate therapy probe and an imaging probe.

As used herein, "cosmetic enhancement" refers to both non-essential and/or essential therapy regimes to human tissue. Cosmetic enhancement therapy includes but is not limited to, for example, mastopexy, treating cellulite, treating blood vessel disorders, and treating stretch marks. As used herein, the phrases "blood vessel disorders", "vascular disorders" and the like include, but are not limited to peripheral vascular deformities such as, for example, varicose veins, spider veins, deep vein disorders, facial hemangiomas or port wine stains, and/or the like.

In accordance with an exemplary embodiment, treatment system 100 is configured for treating a region of interest, by first, imaging of region of interest 106 for localization of the treatment area and surrounding structures, second, delivery of ultrasound energy at a depth, distribution, timing, and energy level to achieve the desired therapeutic effect, and third to monitor the treatment area before, during, and after therapy to plan and assess the results and/or provide feedback.

As to the treatment of region of interest 106, system 100 can be configured to treat one or more the superficial layers of tissue of a breast, e.g., a deep tissue region, such as a region comprising muscular fascia and ligaments, and/or muscle, fat or dermis regions; a deep tissue region that contains a lower part of dermis and proximal protrusions of fat labuli into the dermis; a deep tissue region comprising at least one of spider veins, engorged blood vessels, facial blood vessels, and an occlusion within a blood vessel; and/or the fibrous structures (fascia) within the subcutaneous and/or superficial layers of skin. As used herein, the term dermis refers to any part of the dermis and/or the epidermis.

As to the treatment of one or more of these regions of interest, connective tissue can be permanently tightened by thermal treatment to temperatures about 60 degrees C which causes tissue to shrink immediately by approximately 30% in length. Shrinkage of tissue results in tightening desired for correction of one or more of these regions of interest. Treating through localized heating of regions of stretch marks to temperatures of about 60-90 °C, without significant damage to overlying, underlying, or surrounding tissue, as well as the precise delivery of therapeutic energy to stretch marks and obtaining feedback from the region of interest before, during, and after treatment can be suitably accomplished through treatment system 100. Subsequent tightening of tissue in ROI 106 results in minimization of cosmetic defects in the targeted region in ROI 106 and improved appearance of the overlaying superficial layers of the skin.

Region of interest 106 can vary from one cosmetic enhancement treatment on another. For example, ROI 106 for mastopexy includes for example, the muscular fascia, the Cooper's ligaments, the suspensory ligaments and/or any other ligaments. These are typically about 0.5-2.5 cm deep, and vary in depth and thickness at different locations.

In another embodiment for treating cellulite, by treatment of ROI 106, transducer system 102 may be configured to deliver one or more energy fields to, for example, a lower part of dermis, proximal protrusions of fat labuli into said dermis, and/or a subcutaneous layer. This energy can promote one or more effects, for example, ablation of existing tissue, protein synthesis, the breaking up of fat cell clusters, stretching of the fibrous bonds, enhancement of lymphatic drainage, stimulation of the evacuation of fat decay products, and/or enhanced cell permeability in order to treat cellulite.

In yet another embodiment, ROI 106 for treatment of blood vessel disorders, may be, for example spider veins, engorged blood vessels, facial blood vessels, and/or an occlusion within a blood vessel that is several millimeters in size and a up to 70 mm deep. ROI 106 can also include other blood vessel defects in the face and body.

As to the treatment of stretch marks, ROI 106 can include connective tissue that can be permanently tightened by thermal treatment to temperatures about 60 degrees C which causes tissue to shrink immediately by approximately 30% in length. Shrinkage of tissue results in tightening and reconfiguration desired for correction of stretch marks. Treating through localized heating of regions of stretch marks to temperatures of about 60-90 °C, without significant damage to overlying, underlying, or surrounding tissue, as well as the precise delivery of therapeutic energy to stretch marks and obtaining feedback from the region of interest before, during, and after treatment can be suitably accomplished through treatment system 100. Subsequent tightening of tissue in ROI 106 results in minimization of stretch marks in the targeted region in ROI 106 and improved appearance of the overlaying superficial layers of the skin.

In another embodiment, exemplary transducer system 100 can also be configured for first, imaging and display of region of interest 106 for localization of the treatment area and surrounding structures, second, delivery of focused, unfocused, or defocused ultrasound energy at a depth, distribution, timing, and energy level to achieve the desired therapeutic effect of thermal ablation to treat cellulite, and third to monitor the treatment area and surrounding structures before, during, and after therapy to plan and assess the results and/or provide feedback to control system 102 and/or an operator.

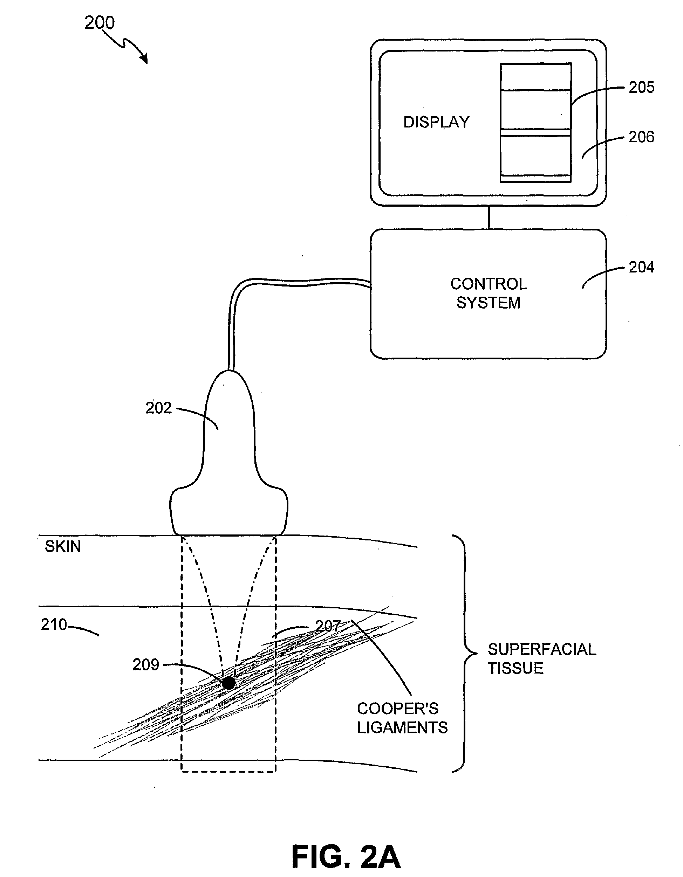

Exemplary cosmetic enhancement treatment systems 100 are configured to provide specific treatment tailored to the specific cosmetic result desired. For example, with reference to an exemplary embodiment illustrated in FIG. 2A, a non-invasive mastopexy system 200 can comprise a therapy transducer system 202, a control system 204 and a display 206 to provide treatment to a region of interest (ROI) 210.

Transducer probe 202 may be configured in a probe arrangement to provide treatment. Transducer probe 202 may also be configured with various mechanical devices to allow for optimal treatment and therapy, for example to provide controlled positioning of ultrasound therapy transducer 202, such as through a non-invasive configuration and through control by control system 204. Further, transducer probe 202 may also be configured for one-dimensional, two-dimensional, and/or annular arrays, and/or for three-dimensional treatment applications such as that described herein.

Exemplary transducer probe 202 can be configured to be suitably controlled and/or operated in various manners. For example, transducer probe 202 may be configured for use within an ultrasound treatment system, an ultrasound imaging system, an ultrasound monitoring system, and/or any combination of an ultrasound treatment, imaging and/or monitoring system including motion control subsystems.

Control system 204 can be configured with one or more subsystems, processors, input devices, displays and/or the like. Display 206 may be configured to image and/or monitor ROI 210 and/or any particular sub-region within ROI 210. Display 206 can be configured for two-dimensional, three-dimensional, real-time, analog, digital and/or any other type of imaging. Exemplary embodiments of both control system 204 and display 206 are described in greater detail herein.

Region of tissue 210 can comprise a superficial layer, such as, for example the epidermis and/or dermis, subcutaneous fat, Cooper ligaments, and/or muscle. Because Cooper's ligaments are typically about 0.5-2.5 cm deep ROI 210 may comprise an extended area of interest. Also, because Cooper's ligaments vary in depth and thickness at different locations, transducer system 200 is configured to facilitate imaging and treatment at different tissue depths and locations.

That is, exemplary transducer system 200, can be configured to provide cross-sectional two-dimensional imaging of a region 207, displayed as an image 205, with a controlled thermal lesion 209, confined within ROI 210. For example, through such spatial and/or temporal control, an exemplary treatment system 200 can enable the regions of thermal injury to possess arbitrary shape and size and allow the tissue to be treated in a controlled manner.

In accordance with an exemplary embodiment, transducer probe 202 can comprise a variable depth transducer including a transduction element having a piezoelectrically active layer, matching layers and/or other materials for generating radiation or acoustical energy. In other words, transducer probe 202 may be configured to operate at moderate frequencies to provide variable depth treatment within ROI 210. Moreover, transducer probe 202 can also be configured as a multi-directional transducer. Various configurations of transducer probe 202 are described in detail herein.

Exemplary ultrasound transducer probe 202 can be configured in various manners to provide various functions. In one embodiment, transducer probe 202 can be configured for generating high acoustic power for treatment purposes, while also providing for good imaging capabilities. For example, to allow for the treatment spot size to be optimally controlled at various treatment depths, an exemplary embodiment of the present invention may comprise a transducer configured into an array of sub-elements, each sub-element configured for processing acoustic waves with a sufficient bandwidth for good axial resolution.

For example, an ultrasound therapy transducer system can be configured for spatial control and/or temporal control by changing the position of transducer, its drive frequency, focal depth, drive amplitude, and timing of the exemplary transducer. In accordance with various exemplary embodiments, transducer probe 202 can be configured for spatial control, such as by changing the distance from transducer probe 202 to a reflecting surface, or changing the angles of energy focused or unfocused to tissue regions 205 and/or 207, and/or configured for temporal control, such as by controlling changes in the frequency, drive amplitude and timing of transducer probe 202 through control system 204. As a result, changes in the location of the treatment region, the shape and size and/or volume of the spot or region of interest, as well as the thermal conditions, can be dynamically controlled versus time.

In addition to the spatial control, control system 204 and/or transducer probe 202 can also be configured for temporal control, such as through adjustment and optimization of drive amplitude levels, frequency/waveform selections, and timing sequences and other energy drive characteristics to control the treatment of tissue. The spatial and/or temporal control can also be facilitated through open-loop and closed-loop feedback arrangements, such as through the monitoring of various positional and temporal characteristics.

In accordance with another exemplary embodiment of the present invention, control system 204 and/or transducer probe 202 can also be configured for generating high acoustic power for treatment purposes, while also providing for good imaging capabilities. For example, to allow for the treatment spot size to be optimally controlled at various treatment depths, an exemplary embodiment of the present invention may comprise a transducer configured into an array of sub-elements, each sub-element configured for processing acoustic waves with a sufficient bandwidth for good axial resolution such as described in U.S. Application Serial No. 10/944,500, *SYSTEMAND METHOD FOR VARIABLE DEPTH ULTRASOUND TREATMENT,* filed 09/16/2004, having at least one common inventor, and hereby incorporated by reference.

In accordance with another aspect of the present invention, exemplary ultrasound therapy treatment system 200 may also be configured to provide therapeutic heating, cooling and/or imaging of a treatment region as well as acoustically monitoring the temperature profile or other tissue parameter monitoring of the treatment region and the general vicinity thereof. For example, in accordance with an exemplary embodiment, ultrasound therapy treatment system 200 may be configured with a dynamic feedback arrangement based on monitoring of temperature or other tissue parameters, and/or based on imaging information to suitably adjust the spatial and/or temporal characteristics of the ultrasound therapy transducer.

In order to facilitate imaging, monitoring, treatment and/or temperature control in ROI 210, control system 204 can be configured with various components and devices. For example, Transducer probe 202 can be configured to provide cross sectional two-dimensional imaging of a region 207, for example as displayed in an image 205 within a display 206, as well as generate a controlled thermal lesion 209, confined proximately to Cooper ligaments and the top of the muscle. Therapeutic ultrasound system 200 can be configured to spare the intervening tissue that contains vital structures, as well as tissue posterior to conformal lesion 209.

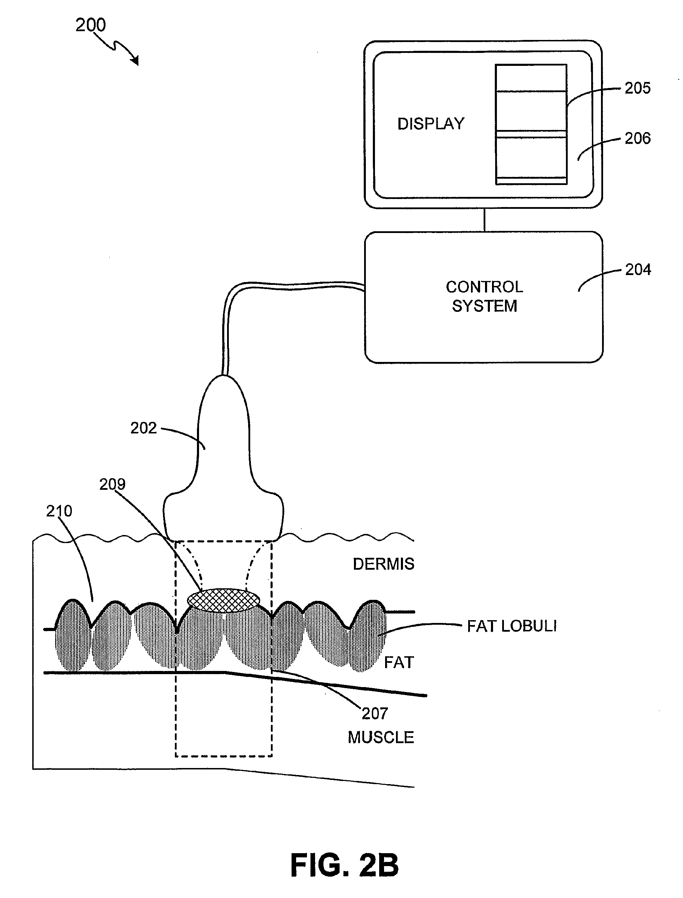

An exemplary cellulite treatment system therapy is illustrated in an exemplary embodiment in FIG. 2B. Cellulite transducer system 200 includes a transducer probe 202 connected to a control system 204, and display 206, in combination may provide therapy, imaging, and/or temperature or other tissue parameters monitoring to region of interest 210.

Region of interest 210, can be comprised of superficial layer (epidermis/dermis) subcutaneous fat, labuli, and muscle. Exemplary transducer system 200, is configured to provide cross-sectional two-dimensional imaging of the region 207, displayed as an image 205, with a controlled thermal lesion 209, confined approximately to proximal portion of fat labuli and lower portion of dermis.

Transducer system 200 may be used to provide a mechanical action of ultrasound to physically break fat cell clusters and stretch the fibrous bonds. This mechanical action will also enhance lymphatic drainage, stimulating the evacuation of fat decay products. That is, the ultrasound may facilitate movement of the muscles and soft tissues within ROI 210, thereby facilitating the loosening of fat deposits and/or the break up of fibrous tissue surrounding fat deposits.

In addition, transducer system 200 can be configured to deliver various therapeutic levels of ultrasound to increase the speed at which fat metabolizes, according to the Arrhenius Law: Y=A • e ^{-B/T}, where Y is the yield of metabolic reaction, A and B are constants, and T is the temperature in degrees Kelvin. In one exemplary embodiment, transducer system 200 is configured to provide various therapeutic levels of ultrasound to increase the speed at which fat metabolizes. That is, according to Arrhenius Law, the yield, Y of a metabolic reaction is a function of temperature, T: Y=A • e ^{-B/T}, where A and B are constants, and T is the temperature in degrees Kelvin. Thus, ultrasound treatment from transducer system 200, ranging from approximately 750 kHz to 20 MHz, can increase the temperature in a treatment area, thereby increasing the metabolic reaction yield for that treatment area.

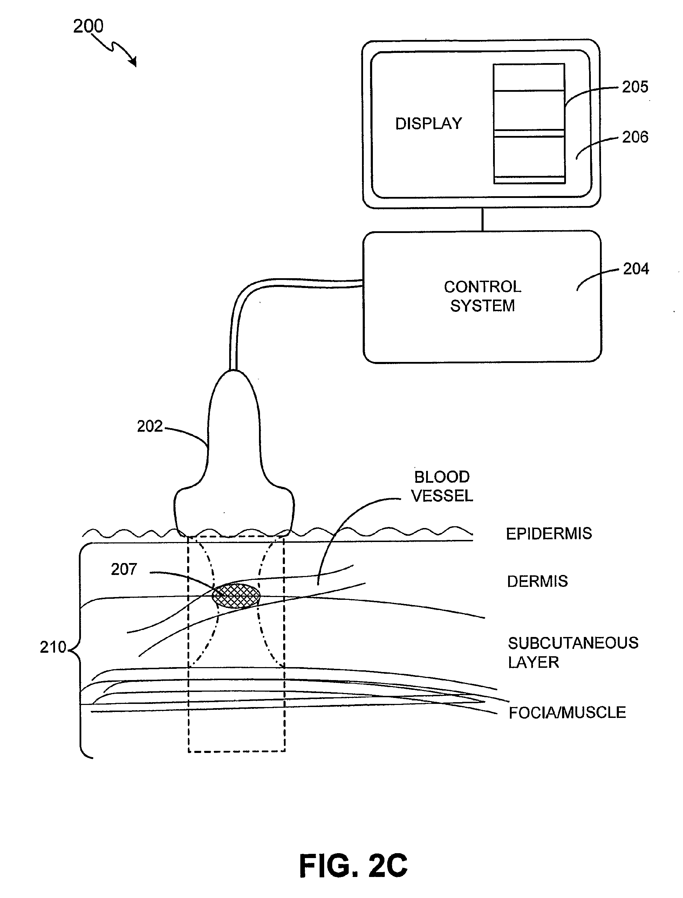

An exemplary blood vessel disorder treatment system is illustrated in an exemplary embodiment in FIG. 2C. Exemplary blood vessel disorder treatment system 200 includes a transducer/probe 202 connected to a control system 204, and display 206, in combination may provide therapy, imaging, and/or temperature or other tissue parameters monitoring to region of interest 210.

Region of interest 210 can comprise any particular vessel or group of vessels and/or any portion within a vessel. Exemplary transducer system 200, is configured to provide cross-sectional two-dimensional imaging of the region 207, displayed as an image 205, with a controlled thermal lesion confined approximately to approximately .1 to 5 mm in diameter in order facilitate ablation of the vessel and approximately 3 to 20 mm in diameter in order facilitate ablation of the vessel. The lesion may be any shape to provide ablation of the blood vessel. For example, spherical, ellipsoid, and/or cigar shaped lesions may be effective for ablation purposes. Methods for treating blood vessels are disclosed further herein.

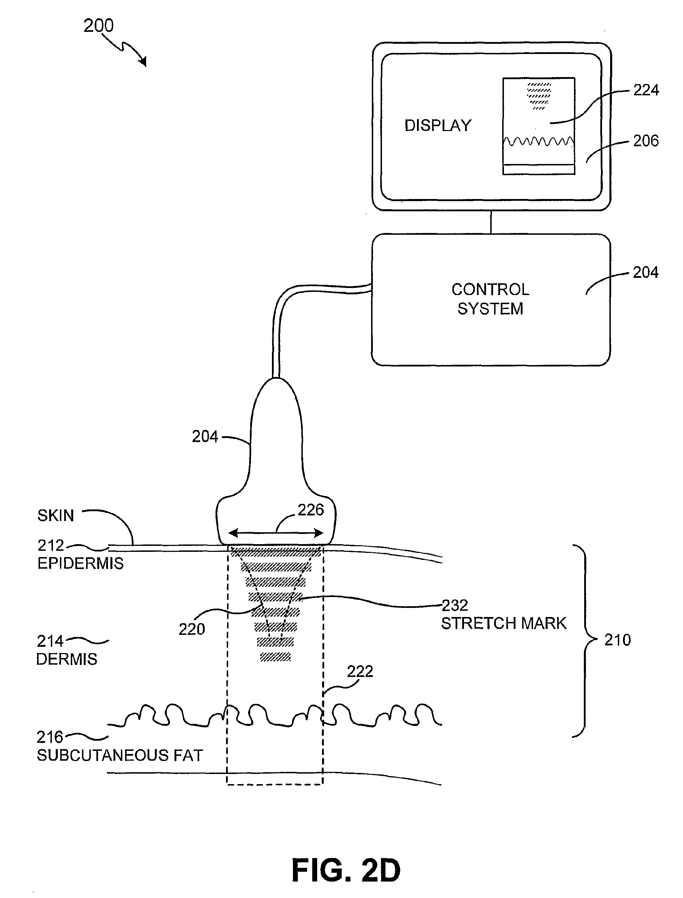

An exemplary cellulite treatment system 200 is illustrated in FIGS. 2D-2F. Exemplary cellulite treatment system 200, with reference to FIG. 2D, comprises an imaging 222 area and display 206 of the region of interest 210 for localization of the treatment area and surrounding structures. Exemplary cellulite treatment system 200 is configured to deliver at least one of focused, unfocused, or defocused ultrasound energy 220 at a depth, distribution, timing, and energy level to achieve the desired therapeutic effect of thermal ablation to treat stretch mark 232, and to monitor the treatment area and surrounding structures before, during, and after therapy to plan and assess the results and/or provide feedback to control system 204 and operator. Exemplary probe 202 and/or transducers can be mechanically and/or electronically scanned 226 to place treatment zones over an extended area, and the treatment depth 220 can be adjusted between a range of approximately 0 to 10 mm, or the maximum depth of the stretch marks or deep dermis.

Region of tissue 210 can comprise a superficial layer, such as, for example the epidermis and/or dermis, subcutaneous fat, and/or muscle. Exemplary transducer system 200, can be configured to provide cross-sectional two-dimensional imaging 222 of ROI 210, displayed as an image 224, with a controlled thermal lesion 220.

In order to deliver energy to ROI 210, transducer probe 202 and/or any other transducers can be mechanically and/or electronically scanned 226 to place treatment zones over an extended area. In one embodiment, a treatment depth 220 can be adjusted between a range of approximately 0 to 10 mm, or the maximum depth of the stretch marks or deep dermis.

In one embodiment, imaging 222 component can comprise a display 206 of ROI 210 to facilitate localization of the treatment area and surrounding structures. Energy 220 may be delivered to ROI 210 using transducer probe 202 configured to deliver focused, unfocused, and/or defocused ultrasound energy 220 at one or more treatment parameters. Various configurations of transducer probe 202 are disclosed herein. As used herein, the phrase "treatment parameters" includes, for example, a depth, distribution, timing, and/or energy level used to achieve a desired therapeutic effect of thermal ablation to treat stretch mark 232.

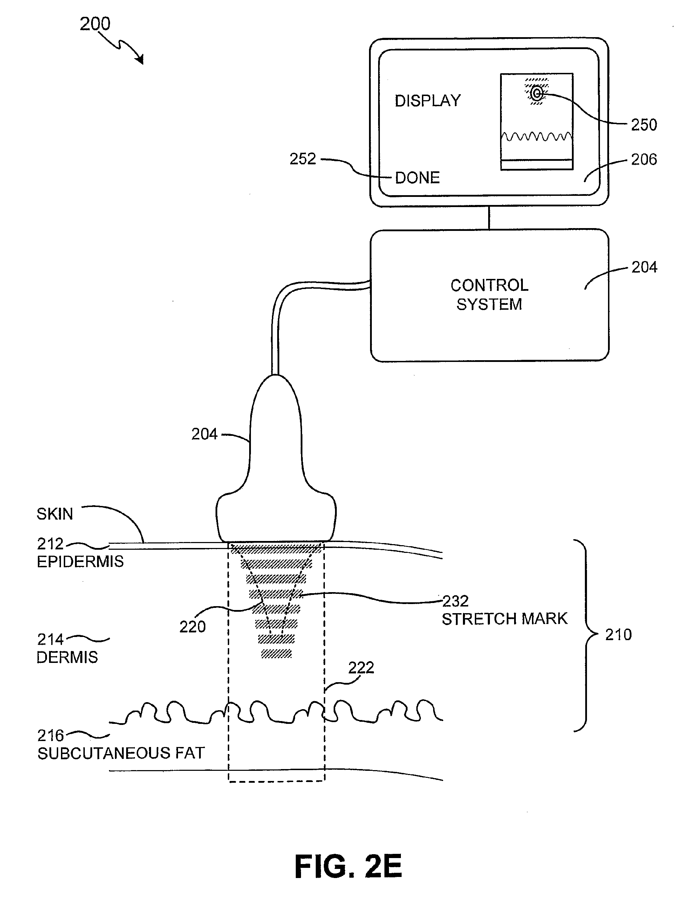

In accordance with another aspect of the present invention, with reference to FIG. 2E, an exemplary cellulite treatment system 200 may be configured monitor the temperature profile or other tissue parameters of the region of interest 210 and/or treatment zone 220, such as attenuation, speed of sound, or mechanical properties such as stiffness and strain, and suitably adjust the spatial and/or temporal characteristics and energy levels of the ultrasound therapy transducer. The results of such monitoring methods may be indicated on display 206 by means of one-, two-, or three-dimensional images of monitoring results 250, or may be as simple as success or fail type indicator 252, or combinations thereof. Additional treatment monitoring methods may be based on one or more of temperature, video, profilometry, and/or stiffness or strain gauges or any other suitable sensing method.

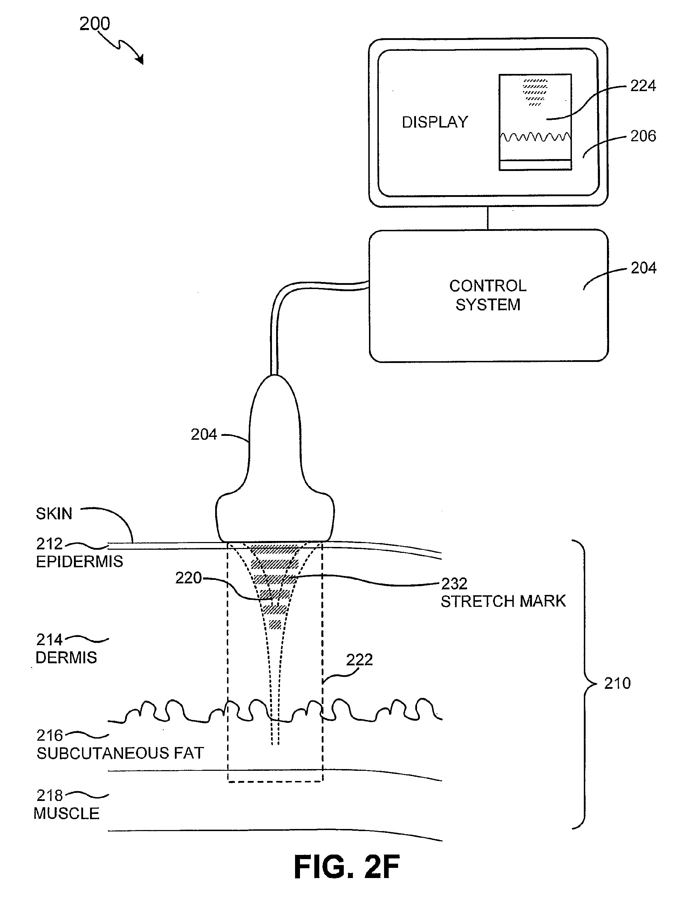

In accordance with another exemplary embodiment, with reference to FIG. 2F, exemplary cellulite treatment system 200 can be configured to provide treatment an expanded treatment region of interest 252, including a combination of tissues, such as subcutaneous fat/adipose tissue 216 and muscle 218, among others. A multiple of such tissues may be treated including stretch marks in combination with at least one of epidermis 212, dermis 214, adipose tissue 216, muscular fascia, muscle 218, hair, glands, and blood vessels within dermis 214, or other tissue of interest. For example, treatment 220 of stretch mark may be performed in combination with treatment of subcutaneous fat 216 by suitable adjustment of the treatment parameters and or transducers in probe 202.

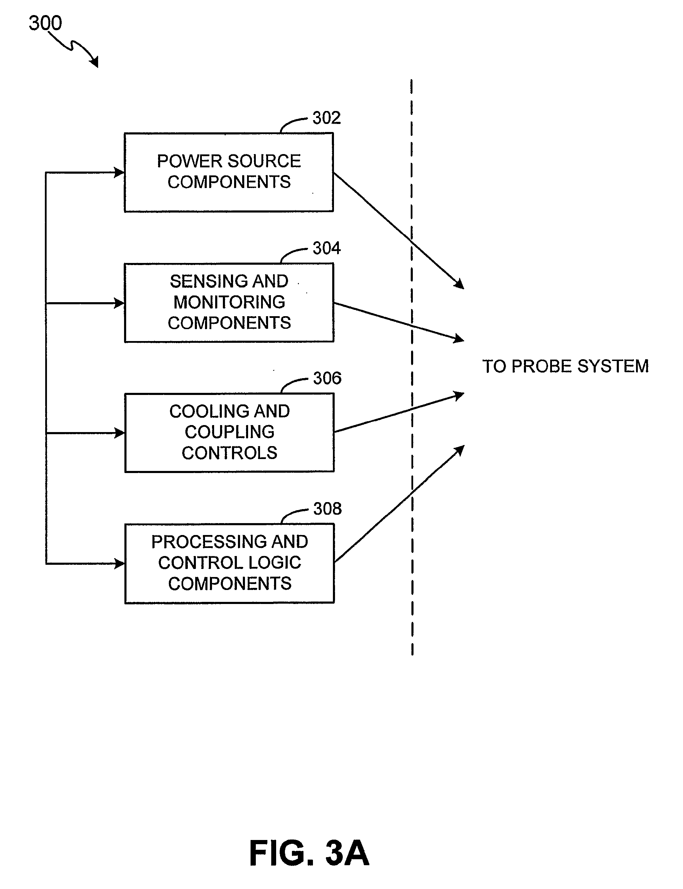

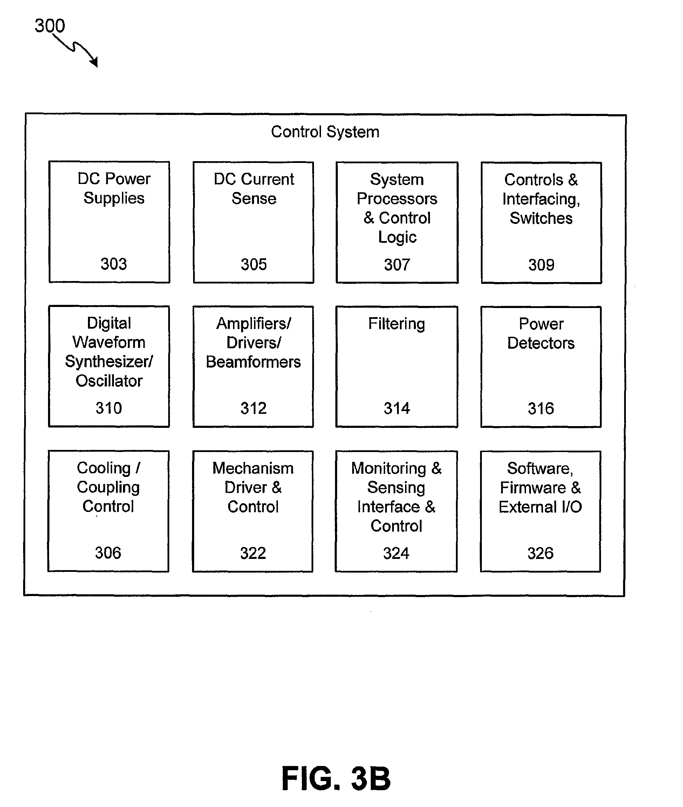

As previously described, control systems 102 and 204 may be configured in various manners with various subsystems and subcomponents. With reference to FIGS. 3A and 3B, in accordance with exemplary embodiments, an exemplary control system 300 can be configured for coordination and control of the entire therapeutic treatment process in accordance with the adjustable settings made by a therapeutic treatment system user. For example, control system 300 can suitably comprise power source components 302, sensing and monitoring components 304, cooling and coupling controls 306, and/or processing and control logic components 308. Control system 300 can be configured and optimized in a variety of ways with more or less subsystems and components to implement the therapeutic system for noninvasive mastopexy, and the embodiment in FIGS. 3A and 3B are merely for illustration purposes.

For example, for power sourcing components 302, control system 300 can comprise one or more direct current (DC) power supplies 303 configured to provide electrical energy for entire control system 300, including power required by a transducer electronic amplifier/driver 312. A DC current sense device 305 can also be provided to confirm the level of power going into amplifiers/drivers 312 for safety and monitoring purposes.

Amplifiers/drivers 312 can comprise multi-channel or single channel power amplifiers and/or drivers. In accordance with an exemplary embodiment for transducer array configurations, amplifiers/drivers 312 can also be configured with a beamformer to facilitate array focusing. An exemplary beamformer can be electrically excited by an oscillator/digitally controlled waveform synthesizer 310 with related switching logic.

The power sourcing components can also include various filtering configurations 314. For example, switchable harmonic filters and/or matching may be used at the output of amplifier/driver 312 to increase the drive efficiency and effectiveness. Power detection components 316 may also be included to confirm appropriate operation and calibration. For example, electric power and other energy detection components 316 may be used to monitor the amount of power going to an exemplary probe system.

Various sensing and monitoring components 304 may also be suitably implemented within control system 300. For example, in accordance with an exemplary embodiment, monitoring, sensing and interface control components 324 may be configured to operate with various motion detection systems implemented within transducer probe 104 to receive and process information such as acoustic or other spatial and temporal information from a region of interest. Sensing and monitoring components can also include various controls, interfacing and switches 309 and/or power detectors 316. Such sensing and monitoring components 304 can facilitate open-loop and/or closed-loop feedback systems within treatment system 100.

For example, in such an open-loop system, a system user can suitably monitor the imaging and or other spatial or temporal parameters and then adjust or modify same to accomplish a particular treatment objective. Instead of, or in combination with open-loop feedback configurations, an exemplary treatment system can comprise a closed-loop feedback system, wherein images and/or spatial/temporal parameters can be suitably monitored within monitoring component to generate signals.

During operation of exemplary treatment system 100, a lesion configuration of a selected size, shape, orientation is determined. Based on that lesion configuration, one or more spatial parameters are selected, along with suitable temporal parameters, the combination of which yields the desired conformal lesion. Operation of the transducer can then be initiated to provide the conformal lesion or lesions. Open and/or closed-loop feedback systems can also be implemented to monitor the spatial and/or temporal characteristics, and/or other tissue parameter monitoring, to further control the conformal lesions.

Cooling/coupling control systems 306 may be provided to remove waste heat from exemplary probe 104, provide a controlled temperature at the superficial tissue interface and deeper, for example into blood and/or tissue, and/or provide acoustic coupling from transducer probe 104 to region-of-interest 106. Such cooling/coupling control systems 306 can also be configured to operate in both open-loop and/or closed-loop feedback arrangements with various coupling and feedback components.

Processing and control logic components 308 can comprise various system processors and digital control logic 307, such as one or more of microcontrollers, microprocessors, field-programmable gate arrays (FPGAs), computer boards, and associated components, including firmware and control software 326, which interfaces to user controls and interfacing circuits as well as input/output circuits and systems for communications, displays, interfacing, storage, documentation, and other useful functions. System software and firmware 326 controls all initialization, timing, level setting, monitoring, safety monitoring, and all other system functions required to accomplish user-defined treatment objectives. Further, various control switches 308 can also be suitably configured to control operation.

An exemplary transducer probe 104 can also be configured in various manners and comprise a number of reusable and/or disposable components and parts in various embodiments to facilitate its operation. For example, transducer probe 104 can be configured within any type of transducer probe housing or arrangement for facilitating the coupling of transducer to a tissue interface, with such housing comprising various shapes, contours and configurations depending on the particular treatment application. For example, in accordance with an exemplary embodiment, transducer probe 104 can be depressed against a tissue interface whereby blood perfusion is partially or wholly cut-off, and tissue flattened in superficial treatment region-of-interest 106. Transducer probe 104 can comprise any type of matching, such as for example, electric matching, which may be electrically switchable; multiplexer circuits and/or aperture/element selection circuits; and/or probe identification devices, to certify probe handle, electric matching, transducer usage history and calibration, such as one or more serial EEPROM (memories). Transducer probe 104 may also comprise cables and connectors; motion mechanisms, motion sensors and encoders; thermal monitoring sensors; and/or user control and status related switches, and indicators such as LEDs. For example, a motion mechanism in probe 104 may be used to controllably create multiple lesions, or sensing of probe motion itself may be used to controllably create multiple lesions and/or stop creation of lesions, e.g. for safety reasons if probe 104 is suddenly jerked or is dropped. In addition, an external motion encoder arm may be used to hold the probe during use, whereby the spatial position and attitude of probe 104 is sent to the control system to help controllably create lesions. Furthermore, other sensing functionality such as profilometers or other imaging modalities may be integrated into the probe in accordance with various exemplary embodiments.

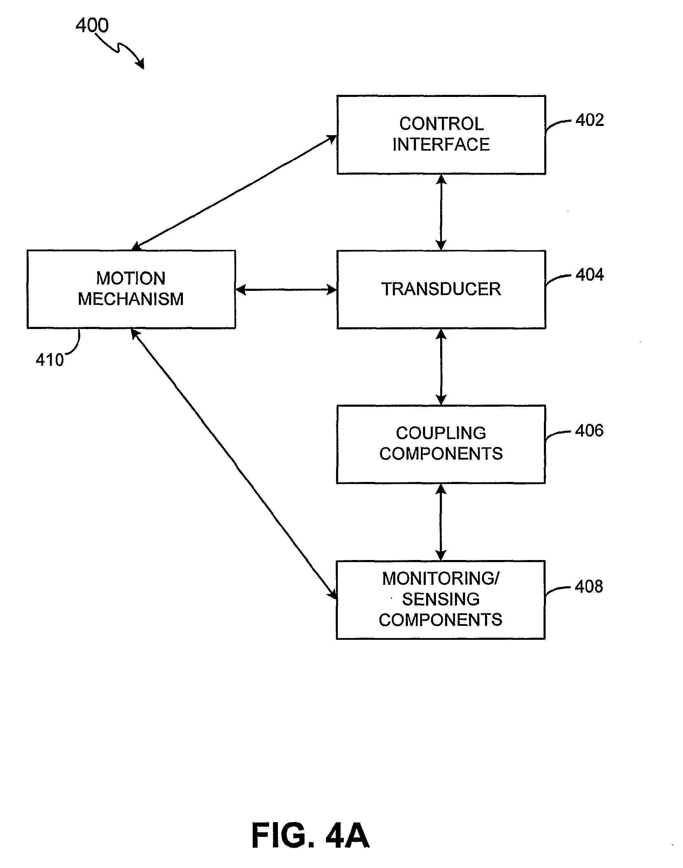

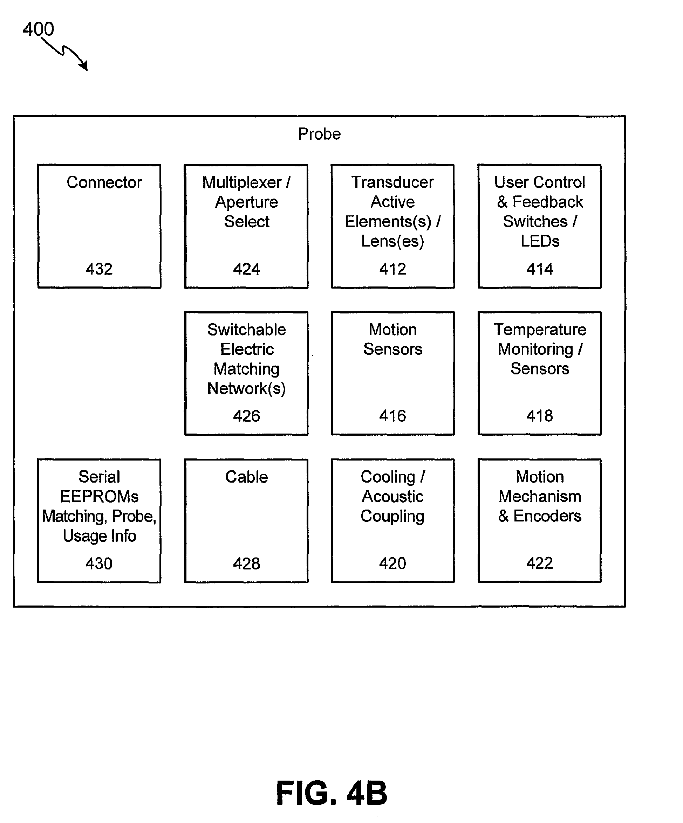

With reference to FIGS. 4A and 4B, in accordance with an exemplary embodiment, a transducer probe 400 can comprise a control interface 402, a transducer 404, coupling components 406, and monitoring/sensing components 408, and/or motion mechanism 410. However, transducer probe 400 can be configured and optimized in a variety of ways with more or less parts and components to provide ultrasound energy for noninvasive mastopexy, and the embodiment in FIGS. 4A and 4B are merely for illustration purposes.
In accordance with an exemplary embodiment of the present invention, transducer probe 400 is configured to deliver energy over varying temporal and/or spatial distributions in order to provide energy effects and initiate responses in a region of interest. These effects can include, for example, thermal, cavitational, hydrodynamic, and resonance induced tissue effects. For example, exemplary transducer probe 400 can be operated under one or more frequency ranges to provide two or more energy effects and initiate one or more responses in the region of interest. In addition, transducer probe 400 can also be configured to deliver planar, defocused and/or focused energy to a region of interest to provide two or more energy effects and to initiate one or more reactions. These responses can include, for example, diathermy, hemostasis, revascularization, angiogenesis, growth of interconnective tissue, tissue reformation, ablation of existing tissue, protein synthesis and/or enhanced cell permeability. These and various other exemplary embodiments for such combined ultrasound treatment, effects and responses are more fully set forth in U.S. Patent Application Serial No. 10/950,112, entitled "METHOD AND SYSTEM FOR COMBINED ULTRASOUND TREATMENT," Filed September 24, 2004 and incorporated herein by reference.

Control interface 402 is configured for interfacing with control system 300 to facilitate control of transducer probe 400. Control interface components 402 can comprise multiplexer/aperture select 424, switchable electric matching networks 426, serial EEPROMs and/or other processing components and matching and probe usage information 430 and interface connectors 432.

Coupling components 406 can comprise various devices to facilitate coupling of transducer probe 400 to a region of interest. For example, coupling components 406 can comprise cooling and acoustic coupling system 420 configured for acoustic coupling of ultrasound energy and signals. Acoustic cooling/coupling system 420 with possible connections such as manifolds may be utilized to couple sound into the region-of-interest, control temperature at the interface and deeper, for example into blood and/or tissue, provide liquid-filled lens focusing, and/or to remove transducer waste heat. Coupling system 420 may facilitate such coupling through use of various coupling mediums, including air and other gases, water and other fluids, gels, solids, and/or any combination thereof, or any other medium that allows for signals to be transmitted between transducer active elements 412 and a region of interest. In addition to providing a coupling function, in accordance with an exemplary embodiment, coupling system 420 can also be configured for providing temperature control during the treatment application. For example, coupling system 420 can be configured for controlled cooling of an interface surface or region between transducer probe 400 and a region of interest and beyond and beyond by suitably controlling the temperature of the coupling medium. The suitable temperature for such coupling medium can be achieved in various manners, and utilize various feedback systems, such as thermocouples, thermistors or any other device or system configured for temperature measurement of a coupling medium. Such controlled cooling can be configured to further facilitate spatial and/or thermal energy control of transducer probe 400.

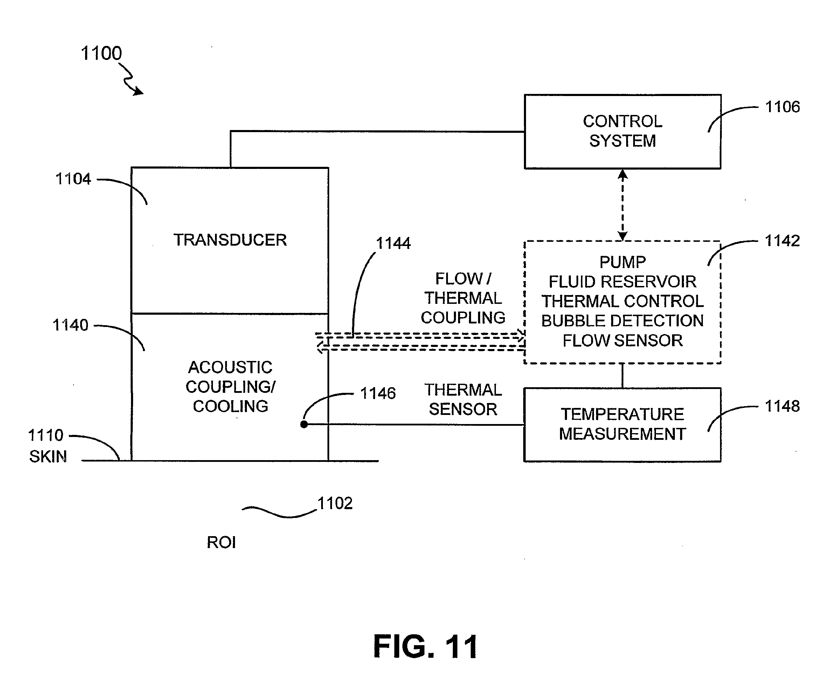

In accordance with an exemplary embodiment, with additional reference to FIG. 11, acoustic coupling and cooling 1140 can be provided to acoustically couple energy and imaging signals from transducer probe 1104 to and from the region of interest 1106, to provide thermal control at the probe to region-of-interest interface 1110 and deeper, for example into blood and/or tissue and deeper, for example into blood and/or tissue, and to remove potential waste heat from the transducer probe at region 1144. Temperature monitoring can be provided at the coupling interface via a thermal sensor 1146 to provide a mechanism of temperature measurement 1148 and control via control system 1102 and a thermal control system 1142. Thermal control may consist of passive cooling such as via heat sinks or natural conduction and convection or via active cooling such as with peltier thermoelectric coolers, refrigerants, or fluid-based systems comprised of pump, fluid reservoir, bubble detection, flow sensor, flow channels/tubing 1144 and thermal control 1142.

Monitoring and sensing components 408 can comprise various motion and/or position sensors 416, temperature monitoring sensors 418, user control and feedback switches 414 and other like components for facilitating control by control system 300, e.g., to facilitate spatial and/or temporal control through open-loop and closed-loop feedback arrangements that monitor various spatial and temporal characteristics.

Motion mechanism 410 can comprise manual operation, mechanical arrangements, or some combination thereof. For example, a motion mechanism 422 can be suitably controlled by control system 300, such as through the use of accelerometers, encoders or other position/orientation devices 416 to determine and enable movement and positions of transducer probe 400. Linear, rotational or variable movement can be facilitated, e.g., those depending on the treatment application and tissue contour surface.

Transducer 404 can comprise one or more transducers configured for producing conformal lesions of thermal injury in superficial human tissue within a region of interest through precise spatial and temporal control of acoustic energy deposition. Transducer 404 can also comprise one or more transduction elements and/or lenses 412. The transduction elements can comprise a piezoelectrically active material, such as lead zirconante titanate (PZT), or any other piezoelectrically active material, such as a piezoelectric ceramic, crystal, plastic, and/or composite materials, as well as lithium niobate, lead titanate, barium titanate, and/or lead metaniobate. In addition to, or instead of, a piezoelectrically active material, transducer 404 can comprise any other materials configured for generating radiation and/or acoustical energy. Transducer 404 can also comprise one or more matching layers configured along with the transduction element such as coupled to the piezoelectrically active material. Acoustic matching layers and/or damping may be employed as necessary to achieve the desired electroacoustic response.

In accordance with an exemplary embodiment, the thickness of the transduction element of transducer 404 can be configured to be uniform. That is, a transduction element 412 can be configured to have a thickness that is substantially the same throughout. In accordance with another exemplary embodiment, the thickness of a transduction element 412 can also be configured to be variable. For example, transduction element(s) 412 of transducer 404 can be configured to have a first thickness selected to provide a center operating frequency of approximately 2 MHz to 50 MHz, such as for imaging applications. Transduction element 412 can also be configured with a second thickness selected to provide a center operating frequency of approximately 7 kHz to 50 MHz, and typically between 1 MHz and 25 MHz for therapy application. Transducer 404 can be configured as a single broadband transducer excited with at least two or more frequencies to provide an adequate output for generating a desired response. Transducer 404 can also be configured as two or more individual transducers, wherein each transducer comprises one or more transduction element. The thickness of the transduction elements can be configured to provide center-operating frequencies in a desired treatment range.

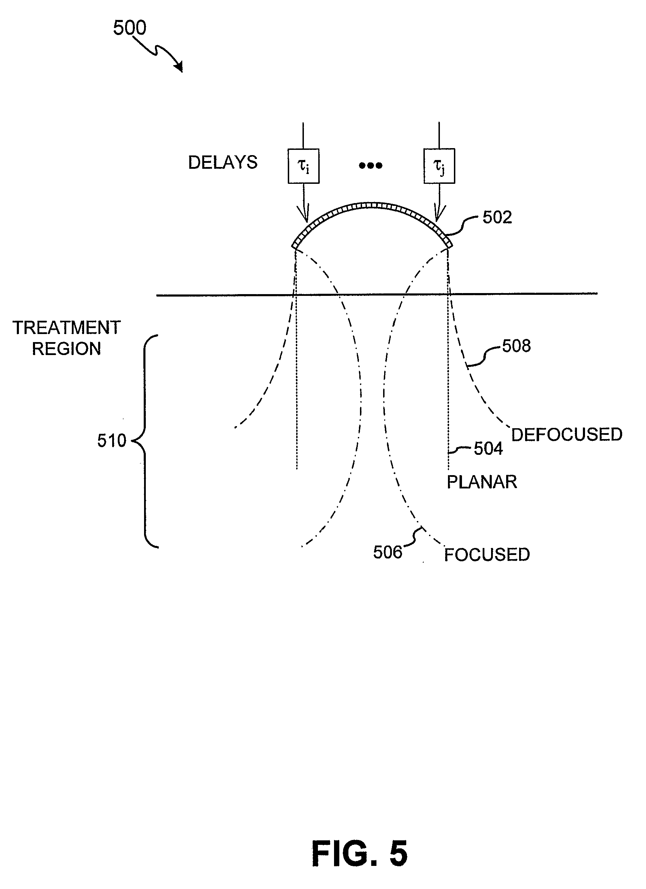

Transducer 404 may be composed of one or more individual transducers in any combination of focused, planar, or unfocused single-element, multi-element, or array transducers, including 1-D, 2-D, and annular arrays; linear, curvilinear, sector, or spherical arrays; spherically, cylindrically, and/or electronically focused, defocused, and/or lensed sources. For example, with reference to an exemplary embodiment depicted in FIG. 5, transducer 500 can be configured as an acoustic array to facilitate phase focusing. That is, transducer 500 can be configured as an array of electronic apertures that may be operated by a variety of phases via variable electronic time delays. By the term "operated," the electronic apertures of transducer 500 may be manipulated, driven, used, and/or configured to produce and/or deliver an energy beam corresponding to the phase variation caused by the electronic time delay. For example, these phase variations can be used to deliver defocused beams, planar beams, and/or focused beams, each of which may be used in combination to achieve different physiological effects in a region of interest 510. Transducer 500 may additionally comprise any software and/or other hardware for generating, producing and or driving a phased aperture array with one or more electronic time delays.

Transducer 500 can also be configured to provide focused treatment to one or more regions of interest using various frequencies. In order to provide focused treatment, transducer 500 can be configured with one or more variable depth devices to facilitate treatment. For example, transducer 500 may be configured with variable depth devices disclosed in U.S. Patent Application 10/944,500, entitled "System and Method for Variable Depth Ultrasound", filed on September 16, 2004, having at least one common inventor and a common Assignee as the present application, and incorporated herein by reference. In addition, transducer 500 can also be configured to treat one or more additional ROI 510 through the enabling of sub-harmonics or pulse-echo imaging, as disclosed in U.S. Patent Application 10/944,499, entitled "Method and System for Ultrasound Treatment with a Multi-directional Transducer", filed on September 16, 2004, having at least one common inventor and a common Assignee as the present application, and also incorporated herein by reference.

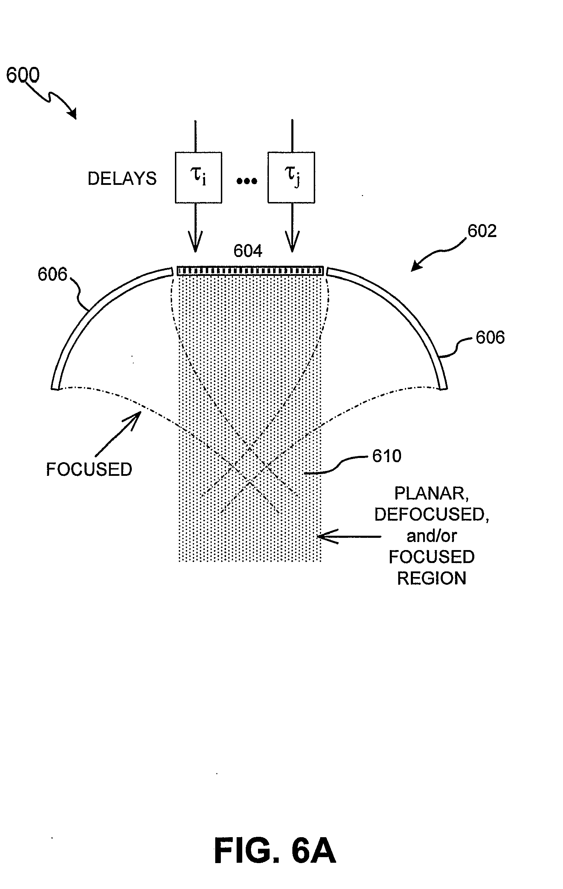

Moreover, any variety of mechanical lenses or variable focus lenses, e.g. liquid-filled lenses, may also be used to focus and or defocus the sound field. For example, with reference to exemplary embodiments depicted in FIGS. 6A and 6B, transducer 600 may also be configured with an electronic focusing array 604 in combination with one or more transduction elements 606 to facilitate increased flexibility in treating ROI 610. Array 604 may be configured in a manner similar to transducer 502. That is, array 604 can be configured as an array of electronic apertures that may be operated by a variety of phases via variable electronic time delays, for example, T₁, T₂...Tⱼ. By the term "operated," the electronic apertures of array 604 may be manipulated, driven, used, and/or configured to produce and/or deliver energy in a manner corresponding to the phase variation caused by the electronic time delay. For example, these phase variations can be used to deliver defocused beams, planar beams, and/or focused beams, each of which may be used in combination to achieve different physiological effects in ROI 610.

Transduction elements 606 may be configured to be concave, convex, and/or planar. For example, in an exemplary embodiment depicted in FIG. 6A, transduction elements 606A are configured to be concave in order to provide focused energy for treatment of ROI 610. Additional embodiments are disclosed in U.S. Patent Application 10/944,500, entitled "Variable Depth Transducer System and Method", and again incorporated herein by reference.

In another exemplary embodiment, depicted in FIG. 6B, transduction elements 606B can be configured to be substantially flat in order to provide substantially uniform energy to ROI 610. While FIGS. 6A and 6B depict exemplary embodiments with transduction elements 604 configured as concave and substantially flat, respectively, transduction elements 604 can be configured to be concave, convex, and/or substantially flat. In addition, transduction elements 604 can be configured to be any combination of concave, convex, and/or substantially flat structures. For example, a first transduction element can be configured to be concave, while a second transduction element can be configured to be substantially flat.

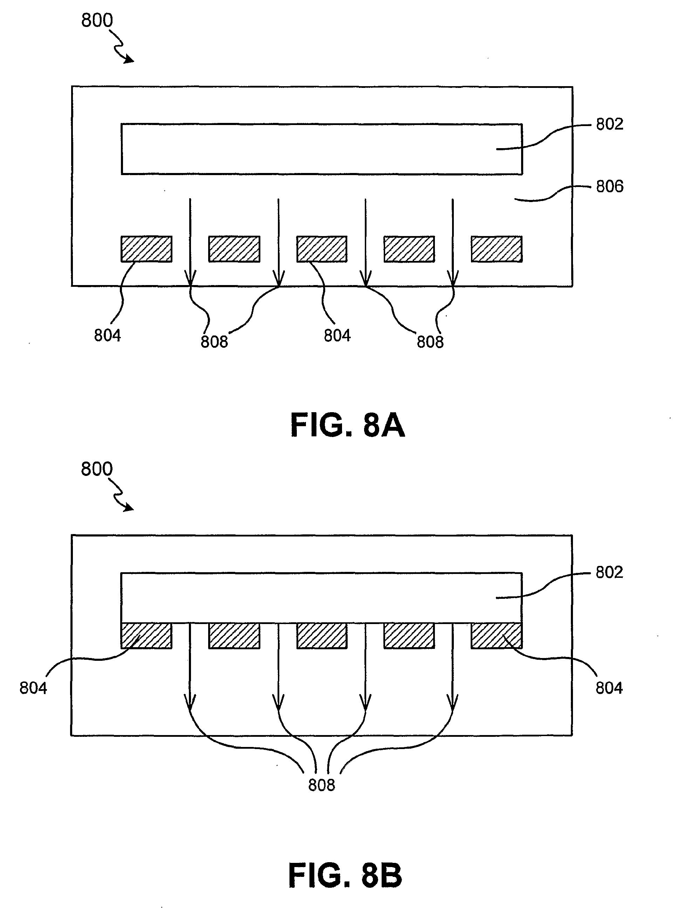

With reference to FIGS. 8A and 8B, transducer 404 can be configured as single-element arrays, wherein a single-element 802, e.g., a transduction element of various structures and materials, can be configured with a plurality of masks 804, such masks comprising ceramic, metal or any other material or structure for masking or altering energy distribution from element 802, creating an array of energy distributions 808. Masks 804 can be coupled directly to element 802 or separated by a standoff 806, such as any suitably solid or liquid material.

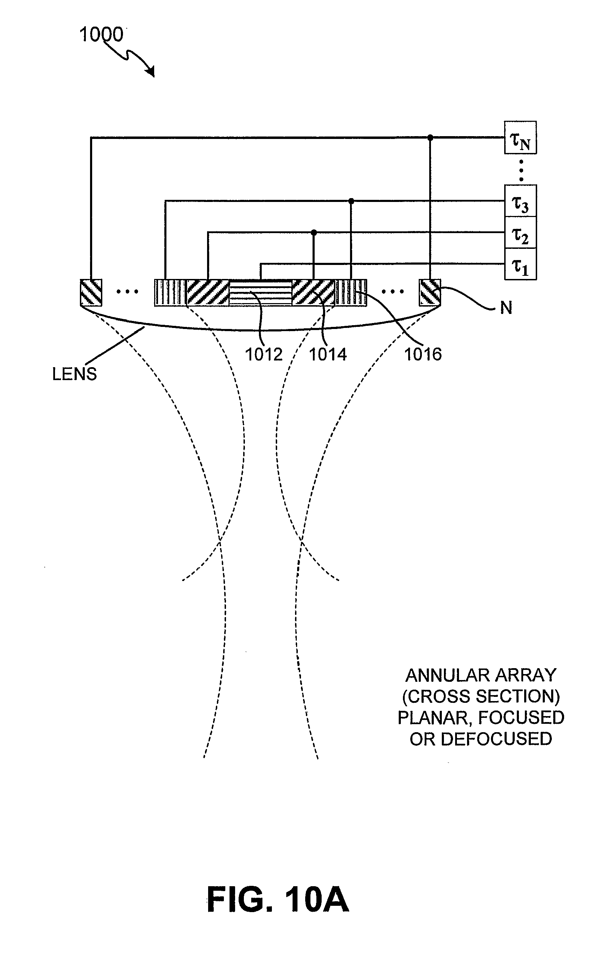

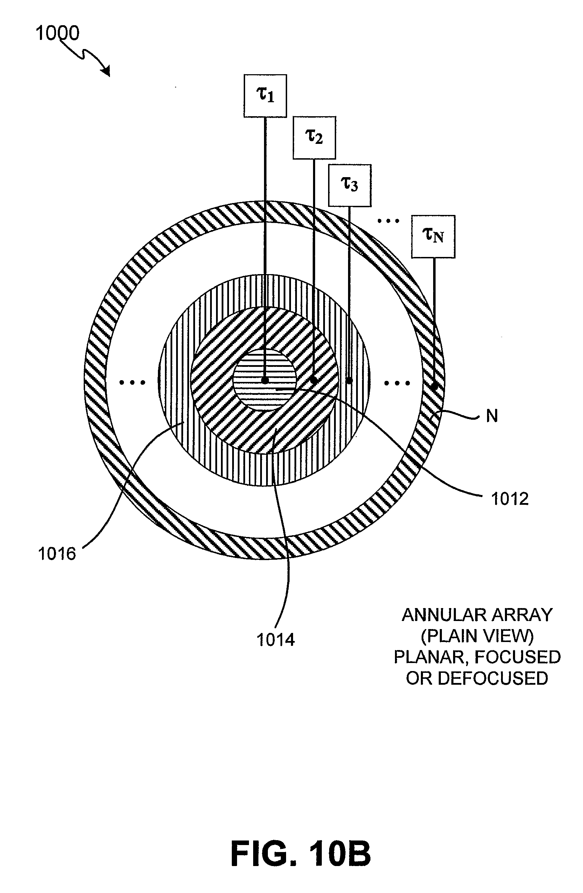

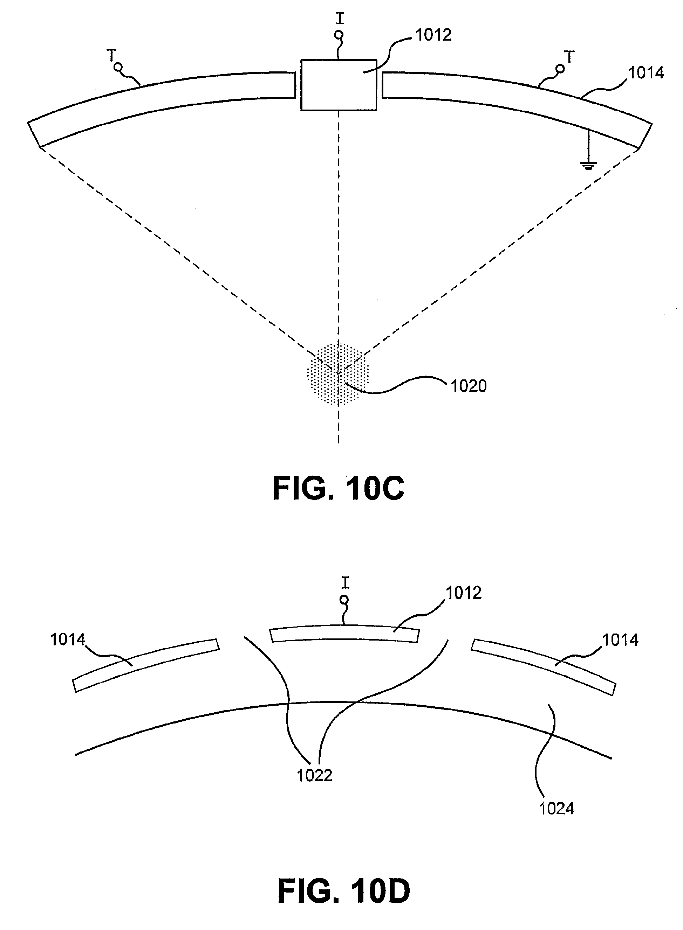

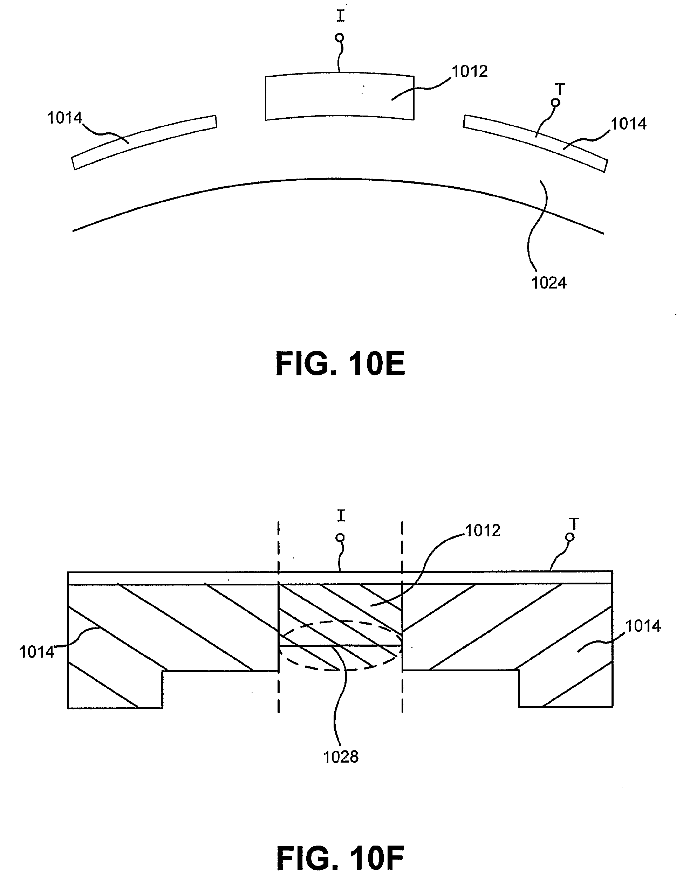

An exemplary transducer 404 can also be configured as an annular array to provide planar, focused and/or defocused acoustical energy. For example, with reference to FIGS. 10A and 10B, in accordance with an exemplary embodiment, an annular array 1000 can comprise a plurality of rings 1012, 1014, 1016 to N. Rings 1012, 1014, 1016 to N can be mechanically and electrically isolated into a set of individual elements, and can create planar, focused, or defocused waves. For example, such waves can be centered on-axis, such as by methods of adjusting corresponding transmit and/or receive delays, τ₁, τ₂, τ₃ ... τ_{N}. An electronic focus can be suitably moved along various depth positions, and can enable variable strength or beam tightness, while an electronic defocus can have varying amounts of defocusing. In accordance with an exemplary embodiment, a lens and/or convex or concave shaped annular array 1000 can also be provided to aid focusing or defocusing such that any time differential delays can be reduced. Movement of annular array 1000 in one, two or three-dimensions, or along any path, such as through use of probes and/or any conventional robotic arm mechanisms, may be implemented to scan and/or treat a volume or any corresponding space within a region of interest.

Transducer 404 can also be configured in other annular or non-array configurations for imaging/therapy functions. For example, with reference to FIGS. 10C-10F, a transducer can comprise an imaging element 1012 configured with therapy element(s) 1014. Elements 1012 and 1014 can comprise a single-transduction element, e.g., a combined imaging/transducer element, or separate elements, can be electrically isolated 1022 within the same transduction element or between separate imaging and therapy elements, and/or can comprise standoff 1024 or other matching layers, or any combination thereof. For example, with particular reference to FIG. 10F, a transducer can comprise an imaging element 1012 having a surface 1028 configured for focusing, defocusing or planar energy distribution, with therapy elements 1014 including a stepped-configuration lens configured for focusing, defocusing, or planar energy distribution.

In accordance with another aspect of the invention, transducer probe 400 may be configured to provide one, two or three-dimensional treatment applications for focusing acoustic energy to one or more regions of interest. For example, as discussed above, transducer probe 400 can be suitably diced to form a one-dimensional array, e.g., a transducer comprising a single array of sub-transduction elements.

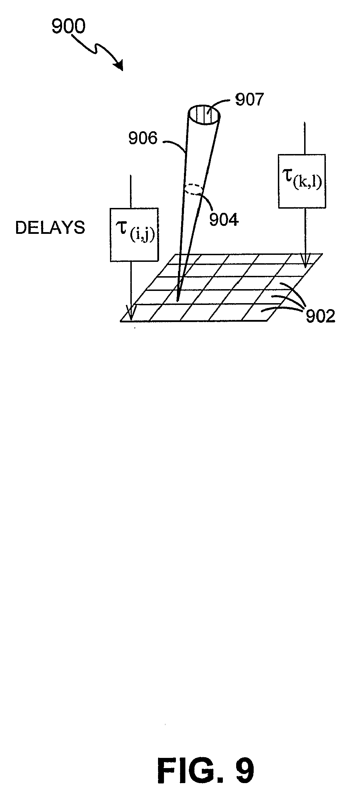

In accordance with another exemplary embodiment, transducer probe 400 may be suitably diced in two-dimensions to form a two-dimensional array. For example, with reference to FIG. 9, an exemplary two-dimensional array 900 can be suitably diced into a plurality of two-dimensional portions 902. Two-dimensional portions 902 can be suitably configured to focus on the treatment region at a certain depth, and thus provide respective slices 904 of the treatment region. As a result, the two-dimensional array 900 can provide a two-dimensional slicing of the image place of a treatment region, thus providing two-dimensional treatment.

In accordance with another exemplary embodiment, transducer probe 400 may be suitably configured to provide three-dimensional treatment. For example, to provide three dimensional treatment of a region of interest, with reference again to FIG. 3, a three-dimensional system can comprise transducer probe 400 configured with an adaptive algorithm, such as, for example, one utilizing three-dimensional graphic software, contained in a control system, such as control system 300. The adaptive algorithm is suitably configured to receive two-dimensional imaging, temperature and/or treatment information relating to the region of interest, process the received information, and then provide corresponding three-dimensional imaging, temperature and/or treatment information.

In accordance with an exemplary embodiment, with reference again to FIG. 9, an exemplary three-dimensional system can comprise a two-dimensional array 900 configured with an adaptive algorithm to suitably receive 904 slices from different image planes of the treatment region, process the received information, and then provide volumetric information 906, e.g., three-dimensional imaging, temperature and/or treatment information. Moreover, after processing the received information with the adaptive algorithm, the two-dimensional array 900 may suitably provide therapeutic heating to the volumetric region 906 as desired.

Alternatively, rather than utilizing an adaptive algorithm, such as three-dimensional software, to provide three-dimensional imaging and/or temperature information, an exemplary three-dimensional system can comprise a single transducer 404 configured within a probe arrangement to operate from various rotational and/or translational positions relative to a target region.

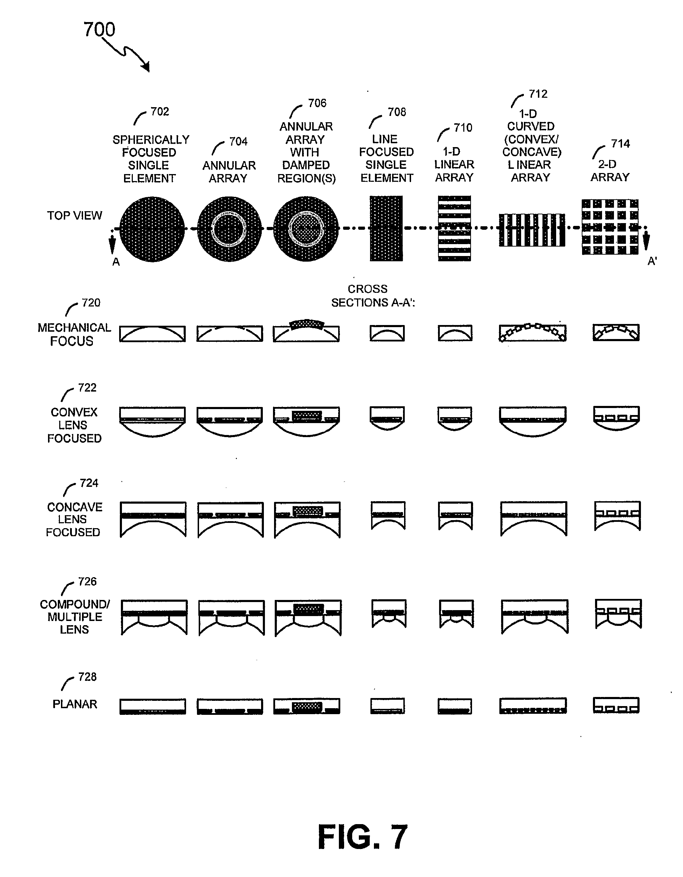

To further illustrate the various structures for transducer 404, with reference to FIG. 7, ultrasound therapy transducer 700 can be configured for a single focus, an array of foci, a locus of foci, a line focus, and/or diffraction patterns. Transducer 700 can also comprise single elements, multiple elements, annular arrays, one-, two-, or three-dimensional arrays, broadband transducers, and/or combinations thereof, with or without lenses, acoustic components, and mechanical and/or electronic focusing. Transducers configured as spherically focused single elements 702, annular arrays 704, annular arrays with damped regions 706, line focused single elements 708, 1-D linear arrays 710, 1-D curvilinear arrays in concave or convex form, with or without elevation focusing, 2-D arrays, and 3-D spatial arrangements of transducers may be used to perform therapy and/or imaging and acoustic monitoring functions. For any transducer configuration, focusing and/or defocusing may be in one plane or two planes via mechanical focus 720, convex lens 722, concave lens 724, compound or multiple lenses 726, planar form 728, or stepped form, such as illustrated in Fig, 10F. Any transducer or combination of transducers may be utilized for treatment. For example, an annular transducer may be used with an outer portion dedicated to therapy and the inner disk dedicated to broadband imaging wherein such imaging transducer and therapy transducer have different acoustic lenses and design, such as illustrated in FIG. 10C-10F.

Various shaped treatment lesions can be produced using the various acoustic lenses and designs in FIGS. 10A-10F. For example, cigar-shaped lesions may be produced from a spherically focused source, and/or planar lesions from a flat source. Concave planar sources and arrays can produce a "V-shaped" or ellipsoidal lesion. Electronic arrays, such as a linear array, can produce defocused, planar, or focused acoustic beams that may be employed to form a wide variety of additional lesion shapes at various depths. An array may be employed alone or in conjunction with one or more planar or focused transducers. Such transducers and arrays in combination produce a very wide range of acoustic fields and their associated benefits. A fixed focus and/or variable focus lens or lenses may be used to further increase treatment flexibility. A convex-shaped lens, with acoustic velocity less than that of superficial tissue, may be utilized, such as a liquid-filled lens, gel-filled or solid gel lens, rubber or composite lens, with adequate power handling capacity; or a concave-shaped, low profile, lens may be utilized and composed of any material or composite with velocity greater than that of tissue. While the structure of transducer source and configuration can facilitate a particular shaped lesion as suggested above, such structures are not limited to those particular shapes as the other spatial parameters, as well as the temporal parameters, can facilitate additional shapes within any transducer structure and source.

Through operation of ultrasound system 100, methods for mastopexy, treating cellulite, treating blood vessel disorders and/or treating cellulite can be realized that can facilitate effective and efficient therapy without creating chronic injury to human tissue. For example, a user may first select one or more transducer probe configurations for treating a region of interest. The user may select any probe configuration described herein. Because the treatment region ranges from approximately 1 mm to 4 cm for mastopexy, from approximately 0 mm to 3.5 cm for treating cellulite, from approximately 0 mm to 7 cm for treating blood vessel disorders and from approximately 0 mm to 1 cm for treating stretch marks, exemplary transducer probes may include, for example, an annular array, a variable depth transducer, a mechanically moveable transducer, a cylindrical-shaped transducer, a linear array, 1-D array, a 2-D array, a curvilinear array, a masked element, and/or any other transducer configuration or combination of transducer configurations described herein. As used herein, the term user may include a person, employee, doctor, nurse, and/or technician, utilizing any hardware and/or software of other control systems.

Once one or more transducers are selected, the user may then image a region of interest in order to plan a treatment protocol. By imaging a region of interest, the user may user the same treatment transducer probe and/or one or more additional transducers to image the region of interest at a high resolution. In one embodiment, the transducer may be configured to facilitate high speed imaging over a large region of interest to enable accurate imaging over a large region of interest. In another embodiment, ultrasound imaging may include the use of Doppler flow monitoring and/or color flow monitoring. In addition other means of imaging such as MRI, X-Ray, PET, infrared or others can be utilized separately or in combination for imaging and feedback of the superficial tissue and the vascular tissue in the region of interest.

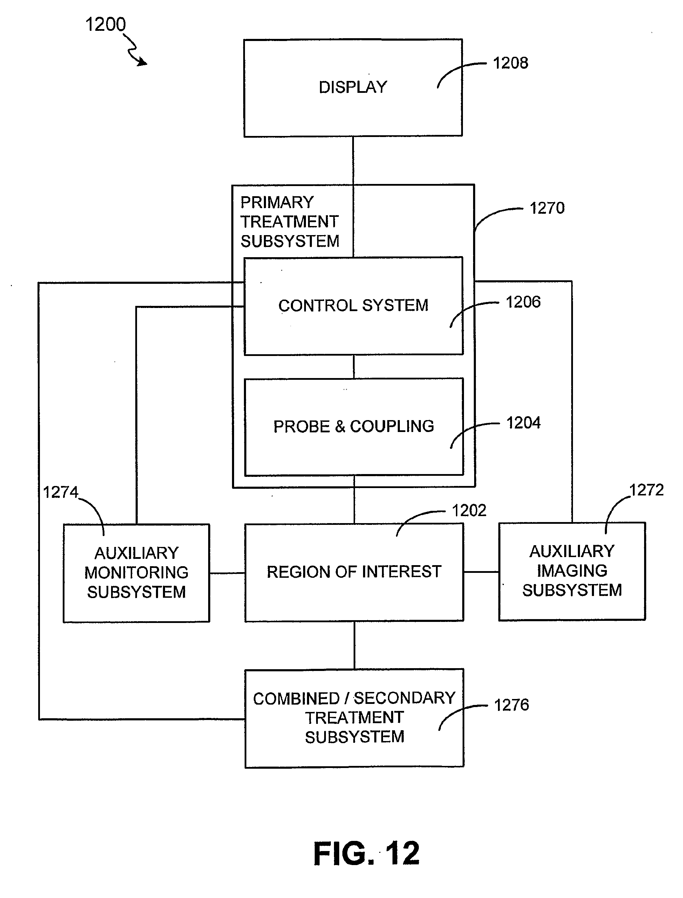

In accordance with another exemplary embodiment, with reference to FIG. 12, an exemplary treatment system 200 can be configured with and/or combined with various auxiliary systems to provide additional functions. For example, an exemplary treatment system 1200 for treating a region of interest 1206 can comprise a control system 1202, a probe 1204, and a display 1208. Treatment system 1200 further comprises an auxiliary imaging modality 1274 and/or auxiliary monitoring modality 1272 may be based upon at least one of photography and other visual optical methods, magnetic resonance imaging (MRI), computed tomography (CT), optical coherence tomography (OCT), electromagnetic, microwave, or radio frequency (RF) methods, positron emission tomography (PET), infrared, ultrasound, acoustic, or any other suitable method of visualization, localization, or monitoring of region-of-interest 1206, including imaging/monitoring enhancements. Such imaging/monitoring enhancement for ultrasound imaging via probe 1204 and control system 1202 could comprise M-mode, persistence, filtering, color, Doppler, and harmonic imaging among others; furthermore an ultrasound treatment system 1270, as a primary source of treatment, may be combined with a secondary source of treatment 1276, including radio frequency (RF), intense pulsed light (IPL), laser, infrared laser, microwave, or any other suitable energy source.

Because the location of Cooper's ligaments, blood vessels, cellulite, and/or stretch marks varies from one patient to another (due to genetics, weight, age, etc.), imaging using a transducer can facilitate tracking the depth of treatment within a patient, imaging the region of interest, and/or determining the length and/or location of one or more treatment targets (i.e, a Cooper's ligament, blood vessel and/or blood vessel occlusion, cellulite, and/or stretch mark) within a patient. This imaging/tracking/determining information can also be used to calculate the optimal ultrasound treatment parameters to facilitate the desired level of cosmetic results.

That is, a user may use the imaging information to facilitate planning of a treatment protocol. To plan the treatment protocol, the user may choose one or more spatial and/or temporal characteristics to provide conformal ultrasound energy to a region of interest. For example, the user may select one or more spatial characteristics to control, including, for example, the use one or more transducers, one or more mechanical and/or electronic focusing mechanisms, one or more transduction elements, one or more placement locations of the transducer relative to the region of interest, one or more feedback systems, one or more mechanical arms, one or more orientations of the transducer, one or more temperatures of treatment, one or more coupling mechanisms and/or the like.

In addition, the user may choose one or more temporal characteristics to control in order to facilitate treatment of the region of interest. For example, the user may select and/or vary the treatment time, frequency, power, energy, amplitude and/or the like in order to facilitate temporal control. For more information on selecting and controlling ultrasound spatial and temporal characteristics, see U.S. Application Serial Number 11/163,148, entitled "Method and System for Controlled Thermal Injury," filed October 6, 2005 and previously incorporated herein by reference.

After planning of a treatment protocol is complete, the treatment protocol can be implemented. That is, a transducer system can be used to deliver ultrasound energy to a treatment region to ablate select tissue in order to facilitate cosmetic treatment. By delivering energy, the transducer may be driven at a select frequency, a phased array may be driven with certain temporal and/or spatial distributions, a transducer may be configured with one or more transduction elements to provide focused, defocused and/or planar energy, and/or the transducer may be configured and/or driven in any other ways hereinafter devised.

In one exemplary embodiment to facilitate mastopexy, energy is delivered at a treatment depth of approximately 1 mm to 4 cm. The energy may range from 1 MHz to about 15 MHz, with typical applications ranging from 2 MHz to 8 MHz. In order to deliver energy in this treatment range, the transducer can be driven at power levels ranging from 10W to 150W or more. Because of the high power and focused treatment that the transducer provides, treatment times for a region of interest can range from 20 milliseconds to 2000 milliseconds or more. Because treatment time and treatment power are interrelated, these variables may differ from one patient to another and/or from one region of interest to another.

In another exemplary embodiment to facilitate cellulite treatment, energy is delivered at a treatment depth of approximately 0 mm to 3.5 cm. The energy may range from 750 kHz to about 10 MHz, with typical applications ranging from 2 MHz to 10 MHz. In order to deliver energy in this treatment range, the transducer can be driven at power levels ranging from 20W to 200W. Because treatment time and treatment power are interrelated, these variables may differ from one patient to another and/or from one region of interest to another.

In one exemplary embodiment to treat blood vessel disorders, ultrasound energy is delivered or deposited at a selective depth to facilitate ablation of a vessel. The ultrasound energy deposition is preferably selectable but not limited to surface of skin tissue ranging from .1 to 5 mm in diameter at a depth of up to 7 mm. The power used to deliver the ultrasound source at one location may range from, for example, about 5 W to about 50 W, and a corresponding source frequency may range from about 2 MHz to about 5 MHz.

In another exemplary embodiment treat blood vessel disorders, ultrasound energy is delivered at a selective depth to facilitate ablation of an occlusion within a vessel. The ultrasound energy deposition is preferably selectable but not limited to surface of skin tissue ranging from 3 to 20 mm in diameter at a depth of up to 70 mm. The power used to deliver the ultrasound source at one location may range from, for example, about 5 W to about 200 W, and a corresponding source frequency may range from about 2 MHz to about 20 MHz. If treatment of the occlusion does not increase blood flow through the region of interest the exemplary transducer system can be used to further ablate the occlusion.

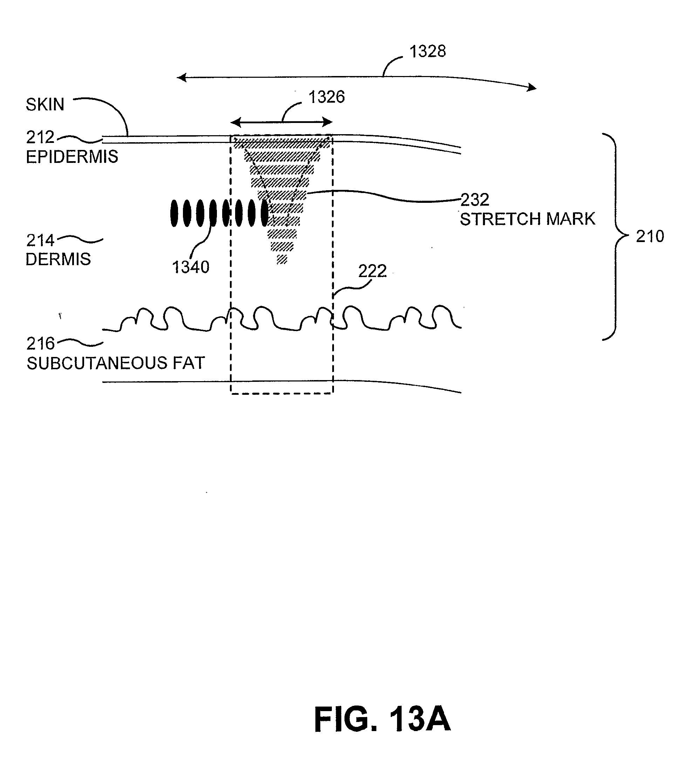

For example and in accordance with another aspect of the present invention for treating stretch marks, and with reference to an exemplary embodiment depicted in FIG. 13A, one or more treated zones 1340 are configured to produce regions of ablation within a treatment volume in spatially defined patterns. These spatially defined patterns include, for example, a discrete locus of treatment spots and/or a one- two- and/or three- dimensional matrix of damage. These spatially defined patterns may be desired rather than heating and destroying an entire volume of the tissue. In such a treatment the surrounding undamaged tissue aids rapid healing and recovery.

Transducer probe 204 and/or any other transducers (not shown) can be mechanically and/or electronically scanned 1326 to extend the treatment zone over a large area, and transducer probe 204 can be further scanned or moved 1328 to further enlarge the treatment zone. The zones of treatment may be placed at depths ranging from approximately 0 to 10 mm, or the maximum depth of the stretch marks or deep dermis. Treatment zones can run parallel and/or perpendicular to stretch marks and/or surrounding tissue to create anisotropic patterns of tissue damage, and/or can cover a two-dimensional matrix extending over the disfiguring pattern of stretch marks.

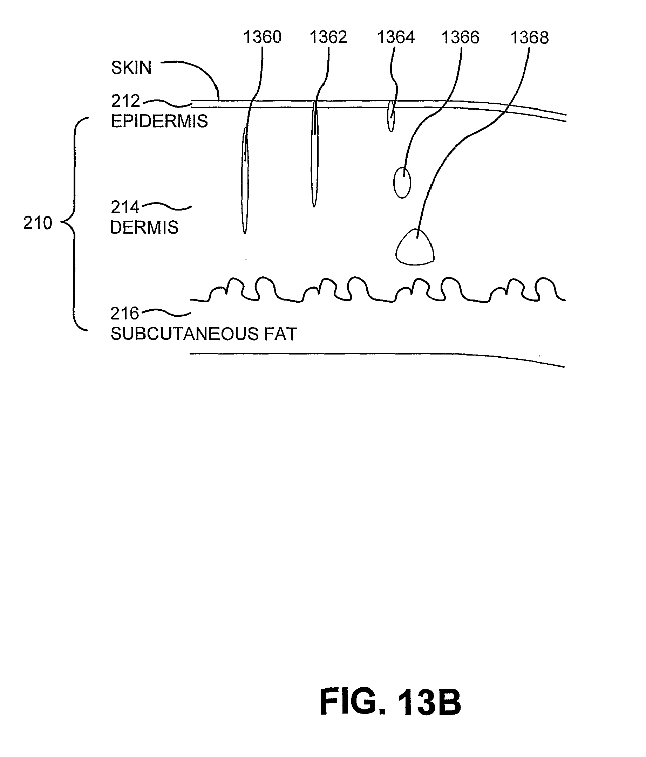

In accordance with another aspect of the present invention for treating stretch marks, and with reference to an exemplary embodiment illustrated in FIG. 13B, a treated zone 1360 may extend throughout regions of the dermis, and may even extend to the epidermis 1362. In addition as treated zone 1360 increases in depth, its cross section may increase from a small size 1364 (about a sub millimeter) in a shallow region near or at the epidermis, to a medium size 1366 (about a sub millimeter to a millimeter) in a middle zone near and/or at the mid dermis, to large size 1368 (about a millimeter) in deep zones near and/or at the deep dermis. Furthermore a single treated zone can have a shape expanding in cross section with depth, and/or be composed of the fusion of several smaller treatment zones. Spacing of treatment zones can be on the order of the treatment zone size or zones or macro-zones may be fused together horizontally.

Once the treatment protocol has been implemented, the region of tissue may have one or more responses in reaction to the treatment. For example, in one embodiment, the tissue responds by causing additional contraction of the Cooper's ligaments and/or other treatment tissues. In another embodiment, the tissue responds by enhancement of lymphatic drainage, evacuation of fat decay products, creation of a thermal injury and/or coagulation of proximal protrusions of fat labuli. As a result there may also be a tightening and/or smoothing of the epidermis. In another embodiment, a blood vessel responds by increased blood flow as an occlusion within the vessel becomes unobstructed. In another embodiment, a blood vessel responds to ablation by disintegrating within the body.

Upon treatment, the steps outlined above can be repeated one or more additional times to provide for optimal treatment results. Different ablation sizes and shapes may affect the recovery time and time between treatments. For example, in general, the larger the surface area of the treatment lesion, the faster the recovery. The series of treatments can also enable the user to tailor additional treatments in response to a patient's responses to the ultrasound treatment.
The present invention has been described above with reference to various exemplary embodiments. However, those skilled in the art will recognize that changes and modifications may be made to the exemplary embodiments without departing from the scope of the present invention. For example, the various operational steps, as well as the components for carrying out the operational steps, may be implemented in alternate ways depending upon the particular application or in consideration of any number of cost functions associated with the operation of the system, e.g., various steps may be deleted, modified, or combined with other steps. These and other changes or modifications are intended to be included within the scope of the present invention, as set forth in the following claims.

## Claims

1. A system for non-invasive cosmetic enhancement **characterized by**:
an ultrasound probe (202) configured to image a ligament within a region of interest (210) comprising a breast and configured to deliver an energy field to at least a portion of the ligament within the region of interest (210) to enhance the breast;
a display unit (206) configured to provide an image (205) of the region of interest (210); and
a controller (204) coupled to and in communication with the ultrasound probe (202) (202) and the display unit (206), the controller (204) configured for controlling delivery of the energy field and for controlling enhancement of the image (205).

2. The system for non-invasive cosmetic enhancement according to claim 1, wherein the energy field is an emission of ultrasound energy.

3. The system for non-invasive cosmetic enhancement according to claim 1, wherein the energy field is configured to tighten the at least a portion of the ligament and to lift the breast.

4. The system for non-invasive cosmetic enhancement according to claim 1, wherein the energy field is configured to generate a thermal lesion (209) in the least a portion of the ligament.

5. The system for non-invasive cosmetic enhancement according to claim 1, further comprising a tissue parameter monitoring subsystem coupled to and in communication with the ultrasound probe (202) and controller (204), the tissue parameter monitoring system configured to monitor tissue in the region of interest (210).

6. A method of non-invasive cosmetic enhancement **characterized by**:
imaging a region of interest (210) in a breast and below a skin surface, the region of interest (210) comprising a muscle and a ligament connected to a portion of breast tissue;
targeting at least a portion of the region of interest (210);
delivering ultrasound energy to the at least a portion of the region of interest (210) at specified depth below the skin surface;
thermally injuring (209) at least a portion of the muscle and the ligament connected to the portion of breast tissue with the ultrasound energy and thereby lifting the breast.

7. The method of non-invasive cosmetic enhancement according to claim 6, further comprising contracting at least one of the muscle and the ligament connected to the portion of breast tissue.

8. A method of non-invasive cosmetic enhancement **characterized by**:
imaging with ultrasound a region of interest (210) of a breast comprising at least one of a ligament, muscular fascia, dermis, a muscle and fat;
delivering ultrasound energy from a transducer probe (202) to at least a portion of the region of interest (210);
tightening at least one of the ligament, the muscular fascia, the dermis, the muscle and the fat in the region of interest (210) with the ultrasound energy; and
monitoring for a modified shape of the breast.

9. A method of non-invasive cosmetic enhancement **characterized by**:
coupling a probe (202) to a region of interest (210) of a breast comprising at least one of epidermis, dermis, subcutaneous fat, a ligament, and a muscle;
providing ultrasound energy from the probe (202);
imaging the region of interest (210);
delivering the ultrasound energy from the probe (202) to a portion of the region of interest (210) to facilitate mastopexy; and
modifying a shape of the breast.

10. The method of non-invasive cosmetic enhancement according to claim 6, 8, or 9, wherein the ligament is a Cooper's ligament.

11. The method of non-invasive cosmetic enhancement according to claim 6, 8, or 9, wherein the ultrasound energy is in the range of 1 MHz to 15 MHz.

12. The method of non-invasive cosmetic enhancement according to claim 6, 8, or 9, wherein the ultrasound energy is in the range of 2 MHz to 8 MHz.

13. The method of non-invasive cosmetic enhancement according to claim 6, 8, or 9, further comprising re-imaging the region of interest (210).

14. The method of non-invasive cosmetic enhancement according to claim 6, 8, or 9, further comprising delivering the ultrasound energy to a second portion of the region of interest (210).
